(19) Europäisches Patentamt
European Patent Office
Office européen des brevets

(11) **EP 1 161 966 B1**

(12) **EUROPÄISCHE PATENTSCHRIFT**

(45) Veröffentlichungstag und Bekanntmachung des Hinweises auf die Patenterteilung:
**06.04.2005 Patentblatt 2005/14**

(51) Int Cl.⁷: **A61N 1/37**, A61N 1/372, A61N 1/39

(21) Anmeldenummer: **01250201.9**

(22) Anmeldetag: **05.06.2001**

(54) **Signalisierungsverfahren für eine implantierbare Herzstimulationsvorrichtung und Herzstimulationsvorrichtung**

Signalling method for an implantable cardiac stimulation device and cardiac stimulation device

Procédé de signalisation pour un dispositif de stimulation cardiaque implantable et dispositif de stimulation cardiaque

(84) Benannte Vertragsstaaten:
**AT BE CH CY DE DK ES FI FR GB GR IE IT LI LU MC NL PT SE TR**

(30) Priorität: **05.06.2000 DE 10028095**

(43) Veröffentlichungstag der Anmeldung:
**12.12.2001 Patentblatt 2001/50**

(73) Patentinhaber: **BIOTRONIK Mess- und Therapiegeräte GmbH & Co Ingenieurbüro Berlin 12359 Berlin (DE)**

(72) Erfinder: **Thong, Tran**
**97229 Portland, Oregon (US)**

(74) Vertreter: **Eisenführ, Speiser & Partner Patentanwälte Rechtsanwälte Spreepalais am Dom Anna-Louisa-Karsch-Strasse 2 10178 Berlin (DE)**

(56) Entgegenhaltungen:
WO-A-86/05698          US-A- 4 788 980
US-A- 4 809 697        US-A- 5 899 928

**Beschreibung**

Die Erfindung betrifft ein Verfahren zur Signalisierung eines internen Zustands einer implantierbaren Herzstimulationsvorrichtung, der durch einen Wert einer Zustandsgröße definiert ist, gemäß dem Oberbegriff des Anspruchs 1. Weiterhin betrifft die Erfindung eine Herzstimulationsvorrichtung gemäß dem Oberbegriff des Anspruchs 10.

Zur externen Erkennung des technischen Zustandes einer implantierbaren Herzstimulationsvorrichtung sind Verfahren und Vorrichtungen bekannt, die es erlauben, die gewünschte Information über den technischen Zustand den von der Vorrichtung abgegebenen Stimulationsimpulsen zu entnehmen. Insbesondere ist aus der Druckschrift US-A-5,899,928 ein Signalisierungsverfahren bekannt, bei dem die zu übermittelnde Information als definierter Zeitabstand zwischen zwei aufeinanderfolgenden Stimulationsimpulsen verschlüsselt ist. Nach Aufnahme eines eine Zustands größe repräsentierenden Messwerts wird dieser durch Vergleich einem von drei Vergleichswertintervallen zugeordnet. Jedem dieser Vergleichsintervalle ist ein definierter zeitlicher Impulsabstand von 500, 600 oder 700 ms zugeordnet. Die Abgabe derartiger Signalimpulse erfolgt nach Detektion eines externen Magnetfeldes. Zur Kenntlichmachung eines nachfolgenden Impulses oder einer nachfolgenden Impulssequenz als Signalimpulse wird zuvor ein nicht näher beschriebener "Synchronisierungsimpuls" erzeugt.

Nachteilig bei diesem Verfahren ist, dass es nur anwendbar ist, wenn der natürliche Herzrhythmus durch die Anwendung einer Schrittmachertherapie völlig außer Kraft gesetzt ist. Ein derartiges Verfahren kann daher nicht bei einem "Demand"-Schrittmacher oder Kardioverter/Defibrillator zur Anwendung kommen, weil hier Stimulationsimpulse nur in Situationen abgegeben werden, in denen der messtechnisch überwachte Zustand des Herzens dies erfordert.

Aufgabe der Erfindung ist es, ein Verfahren sowie eine Herzstimulationsvorrichtung der eingangs genannten Art anzugeben, die die genannten Nachteile nicht aufweisen.

Die Erfindung wird hinsichtlich eines Verfahrens der eingangs genannten Art gelöst durch ein Verfahren zur Signalisierung eines internen Zustands einer implantierbaren Herzstimulationsvorrichtung, der durch einen Wert einer Zustandsgröße definiert ist, umfassend folgende Schritte:

a) Aufnahme eines die Zustandsgröße repräsentierenden Messwerts,

b) Ermitteln des Vorliegens des Zustandes durch Vergleich des Messwerts mit einem Vergleichswert,

c) Messen der Zeitdifferenz gleicher, vorbestimmter Phasen zweier aufeinanderfolgender, periodisch wiederkehrender Ereignisabfolgen,

d) Abgabe mindestens eines Signalimpulses mit einem vorbestimmten, messbaren Impulsparameter, falls der Messwert dem Vergleichswert innerhalb vorgegebener Grenzen entspricht, wobei der Impulsparameter dem Zustand eindeutig zugeordnet ist, indem die Abgabe des Signalimpulses um eine vorbestimmbare und dem Zustand eindeutig zugeordnete Zeitspanne vor dem nächsten Ablauf der gemessenen Zeitdifferenz erfolgt.

Bei dem erfindungsgemäßen Verfahren ist der Zeitpunkt der Abgabe des Signalimpulses an eine vorbestimmte Phase einer periodisch wiederkehrenden Ereignisabfolge gekoppelt. Als Ereignisabfolge wird eine Anzahl von Ereignissen verstanden, die zeitlich nacheinander eintreten. Eine Phase dieser Ereignisabfolge wird durch ein bestimmtes dieser Ereignisse definiert. Der Zeitpunkt des Eintretens der Phase ist der Zeitpunkt des Eintretens dieses bestimmten Ereignisses. Der Zeitabstand zwischen den einzelnen Ereignissen der Ereignisabfolge kann von Periode zu Periode variieren, so dass auch die Dauer der Ereignisabfolge, d.h. einer Periode, von Mal zu Mal veränderlich sein kann. Hierin liegt ein wesentlicher Vorteil des erfindungsgemäßen Verfahrens: eine Kopplung des Zeitpunktes der Signalimpuls-Abgabe an eine vorbestimmte Periodendauer ist nicht erforderlich. Die Durchführung des erfindungsgemäßen Verfahrens ist nicht an vorgegebene Randbedingungen im Hinblick auf die Periodendauer gebunden und somit variabler einsetzbar.

Die aktuelle Periodendauer wird im Verlauf der Durchführung des Verfahrens mit Schritt c) erst kurz vor Abgabe des Signalimpulses gemessen. Hierfür wird die Zeitdifferenz zweiervorbestimmter, gleicher Phasen aufeinanderfolgender Perioden bestimmt. Die spätere dieser beiden Phasen liegt innerhalb der letzten vollständig durchlaufenen Periode vor Abgabe des Signalimpulses. Der Bestimmung des Zeitpunktes der Abgabe des Signalimpulses relativ zur periodischen Ereignisabfolge liegen der gemessene Zeitpunkt des letzten Eintretens der vorbestimmten Phase, die gemessene Periodendauer und der vorbestimmte Zeitversatz zugrunde.

Der zur Signalisierung dienende vorbestimmte, messbare Impulsparameter ist ein Zeitversatz der Abgabe des Signalimpulses gegenüber dem Eintreten der bestimmten Phase der periodischen Ereignisabfolge.

Das Verfahren wird bevorzugt bei einer implantierten Herzstimulationsvorrichtung angewendet. Jedoch ist die Implantation der Vorrichtung in den menschlichen Körper weder ein Teilschritt noch eine notwendige Voraussetzung für die Durchführung des erfindungsgemäßen Verfahrens. Das Verfahren kann ebenso bei einer nicht implantierten Herzstimulationsvorrichtung angewendet werden, beispielsweise zu Testzwecken im Rahmen der Qualitätskontrolle unmittelbar nach der Herstellung oder unmittelbar vor einer Implantation.

Die periodisch wiederkehrende Ereignisabfolge kann bezüglich der implantierbaren Herzstimulationsvorrichtung intern oder extern sein. Die Abgabe des Signalimpulses kann beispielsweise gekoppelt sein an ein internes, also in der Herzstimulationsvorrichtung erzeugtes, periodisches Taktsignal, dessen Frequenz voreinstellbar, also unabhängig von der Dauer einer Herzperiode ist. Diese Frequenz kann auch variabel sein.

In bevorzugten Ausführungsformen der Erfindung wird der Signalimpuls in zeitlichem Bezug zu einer externen, also außerhalb der Herzstimulationsvorrichtung erzeugten, periodisch wiederkehrenden Ereignisabfolge abgegeben. Hierbei handelt es sich beispielsweise um einen externen Ultraschallpuls, der mit Hilfe eines in die Herzstimulationsvorrichtung integrierten Ultraschallsensors detektiert wird. Der Ultraschallsensor erzeugt ein gepulstes, dem Ultraschallpuls entsprechendes elektrisches Signal. Die Zeitdifferenz gleicher, vorbestimmter Phasen zweier aufeinanderfolgender Impulse dieses elektrischen Signals wird gemessen und zur zeitlichen Steuerung der anschließenden Abgabe des Signalimpulses verwendet.

In einer besonders bevorzugten Ausführungsform der Erfindung ist die periodisch wiederkehrende Ereignisabfolge an die Herztätigkeit gekoppelt. Es kann sich bei der Ereignisabfolge beispielsweise um das Eintreten einer definierten Phase eines Messsignals handeln, das den zeitlichen Verlauf der Herztätigkeit widerspiegelt. Hierbei genügt es schon, wenn dieses Messsignal nur den Eintritt einer bestimmten Phase der Herztätigkeit widerspiegelt. Schritt c) des erfindungsgemäßen Verfahrens beinhaltet in diesem Zusammenhang das Messen der Zeitdifferenz gleicher, vorbestimmter Phasen zweier aufeinanderfolgender Herzperioden vor Abgabe des Signalimpulses. Es spielt für die Ausführbarkeit des erfindungsgemäßen Verfahrens keine Rolle, ob die Herztätigkeit stimuliert oder natürlich ist.

Die Messung der Zeitdifferenz gleicher, vorbestimmter Phasen zweier aufeinanderfolgender Herzperioden erfolgt hier allein zur Bestimmung des Zeitpunktes der nachfolgenden Abgabe des Signalimpulses entsprechend dem Schritt d) des Verfahrens. Die gewonnenen Meßdaten werden nicht zur Erstellung einer medizinischen Diagnose der Herztätigkeit verwendet, sondern dienen allein technischen Zwecken.

Selbstverständlich können die mit Schritt c) des Verfahrens gewonnen Messdaten aber für diagnostische Maßnahmen verwendbar sein. Solche diagnostischen Maßnahmen stehen jedoch in keinem Zusammenhang mit dem beanspruchten Verfahren, das allein die Signalisierung eines internen technischen Zustandes einer Herzstimulationsvorrichtung zum Inhalt hat. Ein solcher interner Zustand ist beispielsweise durch einen Wert der Ausgangsspannung eines Akkumulators, oder des elektrischen Widerstandes eines elektrischen Leiters zwischen Bauteilen der Herzstimulationsvorrichtung definiert.

Es versteht sich, dass außerhalb des beanspruchten Verfahrens liegende chirurgische, diagnostische oder therapeutische Maßnahmen weitere nutzbringende Einsatzmöglichkeiten für das erfindungsgemäße Verfahren eröffnen können. Beispielsweise kann das erfindungsgemäße Signalisierungsverfahren in einer Herzstimulationsvorrichtung verwendet werden, um von einem Diagnoseverfahren, das nicht Gegenstand dieser Anmeldung ist, erkannte Herzarrhythmien wie Tachykardie auf eine externe Abfrage hin zu signalisieren.

Auch bekannte therapeutische Maßnahmen erschließen dem erfindungsgemäßen Signalisierungsverfahren Anwendungsgebiete. Beispielsweise kann das erfindungsgemäße Verfahren in einer zur Durchführung einer "on-demand"-Schrittmachertherapie ausgebildeten Herzstimulationsvorrichtung eingesetzt werden. Je nachdem, ob zum Zeitpunkt der Signalisierung eine Schrittmachertherapie durchgeführt wird oder nicht, sind zwei Fälle möglich. In einem Fall wird der Signalimpuls im Anschluß an im Rahmen der Schrittmachertherapie erzeugte Herzstimulationsimpulse abgegeben. Im anderen möglichen Fall erfolgt die Abgabe des Signalimpulses in einer Zeitspanne, in der das Herz ohne Therapieanwendung selbsttätig schlägt. Die Abgabe des Signalimpulses steht in beiden Fällen außerhalb der therapeutischen Maßnahmen. Hier manifestiert sich ein wesentlicher Vorteil des erfindungsgemäßen Verfahrens: es arbeitet unabhängig davon, ob die Herztätigkeit stimuliert oder natürlich ist.

Die Signalimpulse können als elektrische Stromimpulse über eine Stimulationselektrode der Herzstimulationsvorrichtung an den Herzmuskel abgegeben werden. Die Signalimpulse sind von den im Rahmen der Schrittmachertherapie abgegebenen Herzstimulationsimpulsen durch den Zeitpunkt der Impulsabgabe eindeutig unterscheidbar. Dieser Zeitpunkt wird entsprechend den Schritten c) und d) des erfindungsgemäßen Verfahrens bestimmt.

Eine bevorzugte Ausführungsform des erfindungsgemäßen Verfahrens wird gestartet, sobald in der Herzstimulationsvorrichtung ein Auslösesignal vorliegt. Solange das Auslösesignal nicht vorliegt, wird das Verfahren nicht gestartet. Die Erzeugung des Auslösesignals ist vorzugsweise an ein externes Ereignis oder eine externe Zustandsänderung gekoppelt. Beispielsweise kann das Auslösesignal erzeugt werden, wenn ein Magnetfeld oder ein Ultraschallsignal mit vorbestimmten Parametern am Ort der Herzstimulationsvorrichtung detektiert werden. Ein solches Ereignis kann beispielsweise die Empfangsbereitschaft eines Empfängers für den Signalimpuls anzeigen.

Liegt nach der Durchführung des Signalisierungsverfahrens das Auslösesignal weiter vor, wird das Signalisierungsverfahren in einem weiteren Ausführungsbeispiel erneut durchgeführt. So kann auf der Seite des Empfängers der korrekte Empfang des Signalimpulses überprüft werden. Insbesondere neben einem Schrittmachertherapieverfahren fördert die wiederholte Durchführung des Verfahrens die Sicherheit der richtigen Interpretation des Signalimpulses. Denn die Signalisierung des internen Zustands der Herzstimulationsvorrichtung ist durch den Zeitversatz des Signalimpulses im Vergleich mit dem Zeitabstand zweier unmittelbar vorhergehender Herzstimulationsimpulse erkennbar. Ein geringer Zeitversatz kann jedoch messtechnisch schwer auflösbar und erst nach wiederholter Abgabe des Signalimpulses eindeutig zu identifizieren sein.

Ein weiteres Ausführungsbeispiel des erfindungsgemäßen Verfahrens zeichnet sich dadurch aus, dass im Anschluß an die Abgabe des Signalimpulses für eine vorbestimmbare Anzahl nachfolgender Ereignisabfolgen kein Signalimpuls erzeugt wird. Falls das Auslösesignal über eine längere Zeitspanne vorliegt, entsteht auf diese Weise eine periodisch wiederholte Ereignisfolge, die die vorbestimmte Anzahl der periodischen Ereignisse und einen anschließenden Signalimpuls umfaßt. Diese Ereignisfolge wiederholt sich, solange das Auslösesignal vorliegt. Der Nutzen dieses Ausführungsbeispiels liegt darin, dass der Signalimpuls durch seine Wiederholung nach einer regelmäßigen, vorbestimmten Anzahl von Ereignissen leichter erkennbar ist.

Typischerweise beträgt die vorbestimmte Zahl der Ereignisse zwischen den Signalimpulsen fünf. Bevorzugt ist eine externe Programmierbarkeit dieser Zahl vorgesehen, so daß sie beispielsweise variabel zwischen zwei und zwölf voreingestellt werden kann.

Bei einem weiteren Ausführungsbeispiel des erfindungsgemäßen Signalisierungsverfahrens wird eine von der Phase des Herzzyklus abhängige physikalische Größe gemessen. Vorzugsweise wird ein Elektrokardiogramm aufgenommen. Die Aufnahme des Elektrokardiogramms kann mit Hilfe gesonderter Elektroden oder bei einem implantierten Herzstimulationsgerät mit Hilfe der Stimulationselektroden aufgenommen werden. Anhand eines Elektrokardiogramms kann in bekannter Weise die Zeitdifferenz gleicher Phasen zweier aufeinanderfolgender Herzperioden gemessen werden. Die Aufnahme des Elektrokardiogramms dient bei diesem Ausführungsbeispiel - wie oben ausführlich erläutert wurde - keinerlei diagnostischen Zwecken, sondern lediglich der zeitnahen Vorbestimmung des Zeitpunktes der Abgabe des Signalimpulses entsprechend Schritt d) des erfindungsgemäßen Verfahrens.

Gegenwärtig ist der Einsatz des erfindungsgemäßen Verfahrens in einer Ausführungsform bevorzugt, bei der der aufzunehmende Meßwert, der die Zustandsgröße repräsentiert, eine am Ausgang eines in der Herzstimulationsvorrichtung angeordneten Energiespeichers anliegende elektrische Spannung ist. Die Ausgangsspannung des Energiespeichers wird als Maß für seinen Energieinhalt aufgenommen. Sinkt die Ausgangsspannung im Laufe der Zeit auf oder unter einen vorgegebenen Vergleichswert, wird bei Vorliegen des Auslösesignals ein Signalimpuls erzeugt. Auf diese Weise wird das Vorliegen intern registrierter Zustände des Energiespeichers wie "EOL" (end-of-life) oder "ERI" (elective-replacement-indication) einer einfach durchführbaren externen Erkennung zugänglich, beispielsweise bei einer Routinekontrolle in der Praxis eines niedergelassenen Arztes. Die Hinzuziehung eines Spezialisten ist erst erforderlich, wenn das Vorliegen eines dieser Zustände tatsächlich signalisiert wurde.

Hinsichtlich einer Herzstimulationsvorrichtung wird die Aufgabe der Erfindung gelöst durch eine Herzstimulationsvorrichtung mit einem Impulsgenerator, der für die Erzeugung von zur Herzstimulation geeigneten Impulsen ausgebildet ist, mindestens einer mit dem Impulsgenerator verbundenen und zur Abgabe der Impulse ausgebildeten Stimulationselektrode, einem Signalgeber, der mit dem Impulsgenerator verbunden und zur Erzeugung und Abgabe von Impulsauslösesignalen an definierten Zeitpunkten ausgebildet ist, einem Herzphasenmessfühler, der zur Aufnahme eines von der aktuellen Phase des Herzzyklus abhängigen ersten Meßwertes ausgebildet ist, mit Zeitmessmitteln, die mit dem Herzphasenmessfühler verbunden und zur Bestimmung einer Zyklusdauer zwischen dem Eintreten einer vorbestimmten Phase eines definierten ersten Herzzyklus und dem Eintreten der gleichen Phase des unmittelbar nachfolgenden, zweiten Herzzyklus ausgebildet sind, und mit mit den Zeitmessmitteln sowie ausgangsseitig mit dem Signalgeber verbundenen Zeitabstimmmitteln, die zum Vorbestimmen der Zeitspanne zwischen dem Eintreten der vorbestimmten Phase des zweiten Herzzyklus und der nachfolgenden Erzeugung eines Impulsauslösesignals ausgebildet sind.

Die erfindungsgemäße Herzstimulationsvorrichtung weist Zeitabstimmmittel auf. Diese empfangen von den Zeitmessmitteln ein Zyklusdauer-Signal, das der Dauer der zuletzt verstrichenen Herzperiode entspricht.

Unter der Dauer einer Herzperiode ist hierbei die Zeitdifferenz zwischen dem Eintreten einer bestimmten Phase eines Herzzyklus und dem Eintreten der gleichen Phase des unmittelbar nachfolgenden Herzzyklus zu verstehen. Die Bestimmung des Zeitstandes gleicher Phasen aufeinanderfolgender Herzperioden wird beispielsweise anhand des Auftretens der R-Zacken durchgeführt. Am einfachsten ist jedoch die Messung des Zeitintervalls zwischen den Q-Zacken zu realisieren. Die Messung der Periodendauer wird mit dem Erreichen eines vorbestimmten, im vorliegenen Ausführungsbeispiel negativen Spannungs-Schwellwertes gestartet und gestoppt. Diese Methode der Zeitmessung arbeitet zuverlässig sowohl bei natürlicher als auch bei stimulierter Herztätigkeit.

Anhand des Zyklusdauer-Signals wird von den Zeitabstimmmitteln die Zeitspanne vorbestimmt, die vom Ende der gemessenen Herzperiode, d.h. vom letzten Eintreten der bestimmten Phase, bis zur nachfolgenden Erzeugung eines Impulsauslösesignals verstreichen wird.

Die erfindungsgemäße Herzstimulationsvorrichtung ist mit Hilfe der Zeitabstimmmittel in der Lage, nach einer stimulierten wie insbesondere auch nach einer natürlichen, nicht stimulierten Herztätigkeit den Zeitpunkt der anschließenden Erzeugung eines Signalimpulses vorzubestimmen. Ob die Herzperiode, deren Dauer mit Hilfe der Zeitmessmittel bestimmt wurde, durch eine stimulierte oder eine nichtstimulierte, natürliche Herztätigkeit zustandegekommen ist, spielt für die anschließende Signalisierung keine Rolle.

Bei der erfindungsgemäßen Vorrichtung ist ein Signalimpuls ein zur Herzstimulation geeigneter Impuls. Bevorzugt ist der Signalimpuls als ventrikulärer Stimulationsimpuls ausgebildet. Der Signalimpuls gleicht in seinen Impulsparametern wie Spannung und Dauer den im Rahmen einer Schrittmachertherapie üblichen Herzstimulationsimpulse, abgesehen vom Zeitpunkt seiner Abgabe. Der Signalimpuls unterscheidet sich von Stimulationsimpulsen durch seinen Zeitversatz gegenüber der aktuellen Phasenlage der Herztätigkeit.

Der Zeitpunkt der Erzeugung eines Signalimpulses wird bei der erfindungsgemäßen Herzstimulationsvorrichtung erst kurz vor seiner Auslösung vorbestimmt. Direkt nach der zuletzt verstrichenen Herzperiode, deren Dauer durch die Zeitmessmittel beispielsweise anhand des QQ-Zeitintervalls bestimmt wurde, wird der Zeitpunkt der Abgabe des Signalimpulses mit Hilfe der Zeitabstimmmittel berechnet.

Die Zeitspanne zwischen dem Ende der letzten Herzperiode, beispielsweise der letzten Q-Zacke, und dem von den Zeitabstimmmitteln vorbestimmten Zeitpunkt der Abgabe des Signalimpulses weicht von der gemessenen Periodendauer um eine signifikante, messbare Zeitdifferenz ab. Auf diese Weise kann ein mit seinem Abgabezeitpunkt zeitlich auf die augenblickliche Herzperiodendauer abgestimmter, insofern also "synchronisierter" Signalimpuls erzeugt werden. Denn es ist davon auszugehen, dass die Herzperiodendauer sich von einer zur nächsten Periode nur unwesentlich ändert.

Die erfindungsgemäße Vorrichtung ist demnach nicht auf einen festen Stimulationsrhythmus angewiesen, von dem zur Signalisierung abgewichen wird. Vielmehr paßt sie sich mit Hilfe der Zeitabstimmmittel bei der Erzeugung eines Signalimpulses dem augenblicklichen, gegebenenfalls natürlichen Herzrhythmus an. Dies ist vor allem bei Defibrillator-Patienten von großer Bedeutung, da diese auf eine asynchrone Erzeugung von Herzstimulationsimpulsen miteiner Herzarrhythmie reagieren könnten. Die quasi-synchrone Erzeugung eines zur Signalisierung dienenden Herzstimulationsimpulses durch die erfindungsgemäße Vorrichtung vermeidet dieses Risiko.

Die Zeitabstimmmittel sind bei der erfindungsgemäßen Herzstimulationsvorrichtung dazu ausgebildet, unmittelbar nach der Messung der Dauer einer Herzperiode die Abgabe eines Signalimpulses, also eines zeitversetzten Herzstimulationsimpulses zu veranlassen. Bei der Wahl des Zeitversatzes ist zum einen zu berücksichtigen, dass der Zeitversatz extern nachweisbar ist. Der Zeitversatz sollte also ausreichend groß sein, dass Ärzte oder medizinisches Personal durch Auswertung eines EKGs einen zeitversetzten Stimulationsimpuls identifizieren können, und zwarsowohl bei ansonsten natürlicher als auch bei stimulierter Herztätigkeit. Zum anderen ist der Zeitversatz des Signalimpulses so zu wählen, dass das Risiko der Auslösung einer Herzarrhythmie durch den Signalimpuls, wenn nicht vollständig vermieden, so doch besonders gering gehalten wird. Die Ermöglichung einer zeitlichen Abstimmung des Signalimpulses zur aktuellen Phasenlage der Herztätigkeit, stimuliert oder nicht stimuliert, zur Vermeidung einer Stimulation in einer vulnerablen Phase, ist ein wesentlicher Vorteil der Erfindung. Die Signalimpulse werden bevorzugt als verfrühte ventrikuläre Stimulationsimpulse abgegeben.

Für eine in Bezug auf die Herzperiode phasenabgestimmte Abgabe des Signalimpulses sorgen die Zeitabstimmmittel. Hierzu greifen sie auf die von den Zeitmessmitteln erzeugten Messwerte der Periodendauer zu. Da die Zeitmessmittel die Periodendauer anhand des Eintretens einer bestimmten Phase der Herztätigkeit messen, ist mit der Periodendauer auch die Phasenlage der Herztätigkeit bestimmt. Beide Informationen nutzen die Zeitabstimmmittel für eine schonende Abgabe von Signalimpulsen.

Eine nicht pro-arrhythmische Abgabe des Signalimpulses ist sichergestellt, wenn der Zeitpunkt seiner Abgabe die vulnerable Phase des ventrikulären Herzzyklus meidet. Die vulnerable Phase der Herzkammern fällt etwa mit der ansteigenden Flanke der T-Welle des EKG zusammen.

Im folgenden wird den Angaben zum Zeitversatz des Signalimpulses der von den Zeitmessmitteln bestimmte Zeitpunkt des letzten Auftretens der Q-Zacke zugrundegelegt. Es versteht sich jedoch, daß der Wert des Zeitversatzes ein anderer wäre, wenn der Messung der Herzperiodendauer andere Phase der Herztätigkeit zugrunde läge. Wichtig ist die durch die Angaben bestimmte Phasenlage des Zeitpunktes der Abgabe des Signalimpulses relativ zur Herzperiode.

In einer Ausführungsform sind die Zeitabstimmmittel zur Erzeugung des Impulsauslösesignals, das zur Erzeugung des Signalimpulses durch den Impulsgenerator führt, nicht früher als 300 ms vor dem Ablauf der bestimmten Zyklusdauer nach dem Eintreten der vorbestimmten Phase des zweiten Herzzyklus ausgebildet. Dieser Zeitversatz gegenüber dem aktuellen Herzrhythmus ist im Oberflächen-EKG leicht zu erkennen.

In einem besonders bevorzugten Ausführungsbeispiel wird der verfrühte Herzstimulationsimpuls nicht früher als 200 ms vor dem Ablauf der bestimmten Zyklusdauer nach dem Eintreten der vorbestimmten Phase des zweiten Herzzyklus erzeugt. Stimulationsimpulse, die um etwa 100 bis 200 ms verfrüht abgegeben werden, sind ebenfalls leicht im Oberflächen-EKG nachzuweisen. Um die Erkennbarkeit auf dem Oberflächen-EKG sicherzustellen, sollte ein verfrühter Herzstimulationsimpuls nicht später als 50 ms vor dem Ablauf der bestimmten Zyklusdauer nach dem Eintreten der vorbestimmten Phase des zweiten Herzzyklus erzeugt werden.

Eine weitere Ausführungsform der erfindungsgemäßen Vorrichtung weist einen mit den Zeitabstimmmitteln verbundenen Zustandsmessfühler auf, der zur Aufnahme mindestens eines Zustandsmesswertes ausgebildet ist, welcher vom aktuellen Wert einer Zustandsgröße der Vorrichtung abhängt. Es ist insbesondere daran gedacht, dass der Zustandsmessfühler so ausgebildet ist, dass der Zustandsmesswert vom Energieinhalt eines Energiespeichers der Vorrichtung abhängt. Aber auch andere messbare Zustandsgrößen können von einem entsprechend ausgebildeten Zustandsmessfühler erfasst werden, beispielsweise der elektrische Widerstand von mit der Vorrichtung verbundenen Stimulationselektroden. Auch diagnostisch ermittelte Zustände des Herzens des Patienten oder bestimmte pathologische Ereignisse wie beispielsweise eine Herzarrhythmie können von einem Zustandsmessfühler erfasst und von der Herzstimulationsvorrichtung nach außen signalisiert werden.

In einer Ausführungsform ist ein Vergleichswertspeicher zur Aufnahme mindestens eines Vergleichswertes für den Zustandsmesswert vorgesehen. Die Überwachung des Zustandsmesswertes erfolgt dabei vorzugsweise durch erste Vergleichsmittel, die eingangsseitig mit dem Zustandsmessfühler und dem Vergleichswertspeicher sowie ausgangsseitig mit den Zeitabstimmmitteln verbunden sind. Die Vergleichsmittel sind zum Vergleich des Zustandsmesswertes mit dem Vergleichswert sowie zur Erzeugung und Abgabe eines vom Vergleichsergebnis abhängigen Zustandssignals ausgebildet.

Wenn das Zustandssignal anzeigt, dass der Zustandsmesswert nicht innerhalb vorgegebener Grenzen mit dem Vergleichswert übereinstimmt, werden die Zeitabstimmmittel aktiviert. Die Aktivierung der Zeitabstimmmittel erfolgt bei einer Ausführungsform der Erfindung mit Hilfe einer Überwachungseinheit, die eingangsseitig mit den ersten Vergleichsmitteln und ausgangsseitig mit den Zeitabstimmmitteln verbunden ist. Die Überwachungseinheit vergleicht das Zustandssignal mit mindestens einem vorgespeicherten Zustandssollwertintervall und erzeugt ein Wartungssignal, falls das Zustandssignal nicht im Zustandssollwertintervall enthalten ist. Das Wartungssignal wird an die Zeitabstimmmittel übertragen.

Zur Vermeidung der Erzeugung unnötiger Signalimpulse in Situationen, in denen kein externer Empfänger zur Aufnahme des Signalimpulses bereit ist, ist in einer weiteren Ausführungsform der Vorrichtung ein mit den Zeitabstimmmitteln verbundener Aktivierungsmessfühler vorgesehen. Der Aktivierungsmessfühler dient zur Aufnahme von Messwerten (im folgenden Aktivierungsmesswerte) einer Aktivierungsmeßgröße. Dies kann beispielsweise eine magnetische Feldstärke sein, wie sie gängige externe Schrittmacherprüfgeräte erzeugen. Nur wenn der Aktivierungsmeßfühler ein Magnetfeld einer vorbestimmten Feldstärke nachweist, werden bei dieser Ausführungsform die Zeitabstimmmittel aktiviert.

Die Auswertung der vom Aktivierungsmessfühler aufgenommenen Messwerte wird in einer weiteren Ausführungsform mit Hilfe von zweiten Vergleichsmitteln vorgenommen, die ausgangsseitig mit der Überwachungseinheit verbunden ist. Die zweiten Vergleichsmitte greifen auf einen Musterwertspeicher zu. In dem Musterwertspeicher können Muster-Aktivierungswerte abgelegt werden. Hierbei handelt es sich um Aktivierungsmesswerte mit bestimmten Parametern, denen die Bedeutung der Empfangsbereitschaft eines externen Empfängers für den Signalimpuls zugeordnet ist. Die zweiten Vergleichsmittel vergleichen den aktuellen Aktivierungsmesswert mit dem im Musterwertspeicher enthaltenen Muster-Aktivierungswert. Im Falle der Übereinstimmung des Aktivierungsmesswertes mit dem Muster-Aktivierungswert erzeugen die Vergleichsmittel ein Auslösesignal, das von der Überwachungseinheit empfangen wird.

Bei diesem Ausführungsbeispiel erzeugt die Überwachungseinheit ein Wartungssignal nur dann, wenn der Zustandsmesswert außerhalb seines Normwertebereichs liegt und zugleich der Aktivierungsmesswert anzeigt, dass der zu erzeugende Signalimpuls von einem externen Empfänger detektiert werden kann.

Der Herzphasenmessfühler ist bei einem Ausführungsbeispiel zum Aufnehmen eines von der elektrischen Polarisation des Myokards abhängigen Messsignals, vorzugsweise zur Aufnahme eines Elektrokardiogramms ausgebildet. Der Herzphasenmessfühler kann eigens vorgesehen sein, also beispielsweise in Form einer oder mehrerer implantierter Ableitungselektroden. Wo möglich sind jedoch bevorzugt die Stimulationselektroden zugleich als Herzphasenmessfühler ausgebildet, können also sowohl zur Stimulation des Herzens als auch zur Aufnahme eines EKGs verwendet werden.

Eine regelmäßige Abfolge von normalen und zeitversetzten Ereignissen erleichtert die Identifizierung des von der Stimulationsvorrichtung abgegebenen Signalimpulses bei der Auswertung eines Oberflächen-EKGs. In einem hierfür ausgebildeten Ausführungsbeispiel weist die erfindungsgemäße Vorrichtung mit den Zeitabstimmmitteln verbundene Schrittmachersteuermittel auf, die zur wahlweisen Erzeugung eines Aktivierungs- oder eines Deaktivierungssignals zur Aktivierung bzw. Deaktivierung des Betriebs der Zeitabstimmmittel ausgebildet sind. Die Schrittmacher-Steuermittel sind vorzugsweise mit dem Herzphasenmessfühler verbunden und zur Aktivierung oder Deaktivierung des Betriebs der Zeitabstimmmittel für eine vorbestimmbare Anzahl von Herzzyklen ausgebildet. Die Anzahl der Herzzyklen nach einem Signalimpuls wird durch Auswertung des vom Herzphasenmessfühler empfangenen Messsignals bestimmt.

Die Schrittmacher-Steuermittel sind in einer bevorzugten Ausführungsform zusätzlich zur Aktivierung des Betriebs der Zeitabstimmmittel für die Dauer lediglich eines Herzzyklus und zur unmittelbar anschließenden Deaktivierung ausgebildet. Damit wird sichergestellt, dass die verfrühten Herzstimulationsimpulse isoliert sind, d.h., im Anschluss an und gefolgt von einer vorbestimmten Anzahl natürlicher Herzperioden oder regelmäßig stimulierte Herzstimulationsimpulse abgegeben werden. Dies der Schonung des Patienten, da nach Erzeugung eines Signalimpulses sofort zur natürlichen Herztätigkeit oder zur regelmäßigen Stimulation zurückgekehrt wird. Die Dauer der Deaktivierung des Betriebs der Zeitabstimmittel liegt bevorzugt zwischen mindestens zwei und höchstens fünfzehn Herzzyklen. Die Anzahl der Herzzyklen zwischen zwei Signalimpulsen ist in einem Ausführungsbeispiel durch Programmierung der Schrittmachersteuermittel von extern veränderbar.

Bei einem anderen Ausführungsbeispiel der Erfindung ist den Schrittmacher-Steuermitteln eine mit dem Zeitmessmitteln verbundene Herzüberwachungseinheit vorgeschaltet, die zum Vergleich der jeweils gemessenen Periodendauer mit mindestens einem Zyklusdauer-Sollwertintervall ausgebildet ist, das in einem Zyklussollwertspeicher enthalten ist. Die Herzüberwachungseinheit empfängt das Messsignal der Zeitmessmittel, bestimmt die Periodendauer und vergleicht sie mit dem Periodendauer-Sollwertintervall. Für den Fall des Vorliegens einer Abweichung der gemessenen Periodendauer vom Periodendauer-Sollwertintervall ist die Herzüberwachungseinheit in einem weiteren Ausführungsbeispiel zur Erzeugung eines vom Wert der Abweichung abhängigen Therapiewarnsignals ausgebildet.

Vorzugsweise sind die Schrittmacher-Steuermittel zur Erzeugung und Übermittlung des Deaktivierungssignals an die Zeitabstimmmittel ausgebildet, sobald sie ein Therapiewarnsignal von der Herzüberwachungseinheit her empfangen. Auf diese Weise wird die Abgabe verfrühter Herzstimulationsimpulse in solchen Situationen vermieden, in denen der Herzrhythmus von Normwerten abweicht und verfrühte Herzstimulationsimpulse eine Arrhythmie auslösen oder verstärken könnten.

Die Schrittmacher-Steuermittel sind bei einem weiteren Ausführungsbeispiel zur Blockierung der Erzeugung eines Aktivierungssignals für eine definierte Zeitspanne im Falle des Empfangs eines Therapiewarnsignals ausgebildet. Auf diese Weise wird sichergestellt, dass eine primär erforderliche Schrittmachertherapie nicht durch die Abgabe verfrühter Stimulationsimpulse unterbrochen wird.

Die erfindungsgemäße Herzstimulationsvorrichtung hat gegenüber den vorbekannten Lösungen den Vorteil, dass sie in vielen verschiedenen Ausführungsformen realisierbar ist. Ihre besonderen Vorteile können insbesondere bei einer Ausbildung als "on-demand"-Schrittmacher oder integrierter Kardioverter-Defibrillator (ICD) zur Wirkung gelangen. Eine Ausführungsform der Erfindung ist als ICD ausgebildet und weist eine Kardioversionseinheit auf, die zur Erzeugung und Abgabe von zur Kardioversion oderzurDefibrillation geeigneten Stimulationsimpulsen andefinierten Zeitpunkten ausgebildet ist. Die Kardioversionseinheit ist mit den Schrittmacher-Steuermitteln verbunden, die die Aktivierung oder Deaktivierung der Kardioversionseinheit kontrollieren.

Eine weitere Ausführungsform der Erfindung weist zusätzlich für die Durchführung einer Schrittmachertherapie eine eingangsseitig mit dem Herzphasenmessfühler und ausgangsseitig mit dem Signalgeber verbundene Triggereinheit auf, die zur Erzeugung und Abgabe von Herzstimulationsimpulsen an definierten Zeitpunkten im Rahmen einer Schrittmachertherapie ausgebildet ist. Bei dieser Ausführungsform übernehmen die Schrittmacher-Steuermittel vorzugsweise die Koordinierung zwischen Schrittmachertherapie und Signalisierung. Die Schrittmacher-Steuermittel sind mit der Triggereinheit und mit den Zeitabstimmmitteln verbunden und zur Erzeugung und Abgabe mindestens eines Aktivierungs- oder Deaktivierungssignals an die Triggereinheit oder die Zeitabstimmmittel ausgebildet. Bei Abgabe eines Aktivierungssignals an die Schrittmachereinheit erfolgt auch die Abgabe eines Deaktivierungssignals an die Zeitabstimmmittel. Vice versa erfolgt bei Abgabe eines Aktivierungssignals an die Zeitabstimmmittel auch die Abgabe eines Deaktivierungssignals an die Triggereinheit. Die Abgabe von Aktivierungs- und Deaktivierungssignalen kann der Einfachheit halber auch durch eine von den Schrittmacher-Steuermitteln gesteuerte Umschaltvorrichtung ersetzt werden, die wahlweise die Zeitabstimmmittel oder die Triggereinheit aktiviert. Die Triggereinheit ist hiebei zu den Zeitabstimmmitteln parallel geschaltet. Der Signalgeber wird also entweder mit Hilfe über die Zeitabstimmmittel oder über die Triggereinheit angesteuert.

Die Signalisierungsimpulse werden bei einer Vorrichtung mit integrierten Schrittmacherfunktionen der Einfachheit halber ebenfalls von der Schrittmachereinheit erzeugt, so daß hier auf einen eigens für die Erzeugung von Signalimpulsen ausgebildeten Signalimpulsgenerator verzichtet werden kann.

Weitere Ausführungsbeispiele des erfindungsgemäßen Verfahrens und der erfindungsgemäßen Vorrichtung werden im folgenden anhand der Zeichnungen beschrieben. Darin zeigt:

Figur 1    ein Flussdiagramm eines Ausführungsbeispiels des erfindungsgemässen Verfahrens,

Figur 2    in schematischer Darstellung ein während der Durchführung des erfindungsgemäßen Verfahrens aufgezeichnetes EKG-Signal,

Figur 3    ein Blockdiagramm eines Ausführungsbeispiels der erfindungsgemässen Herzstimulationsvorrichtung und

Figur 4    ein Blockdiagramm der Steuereinheit des Ausführungsbeispiels nach Figur 3.

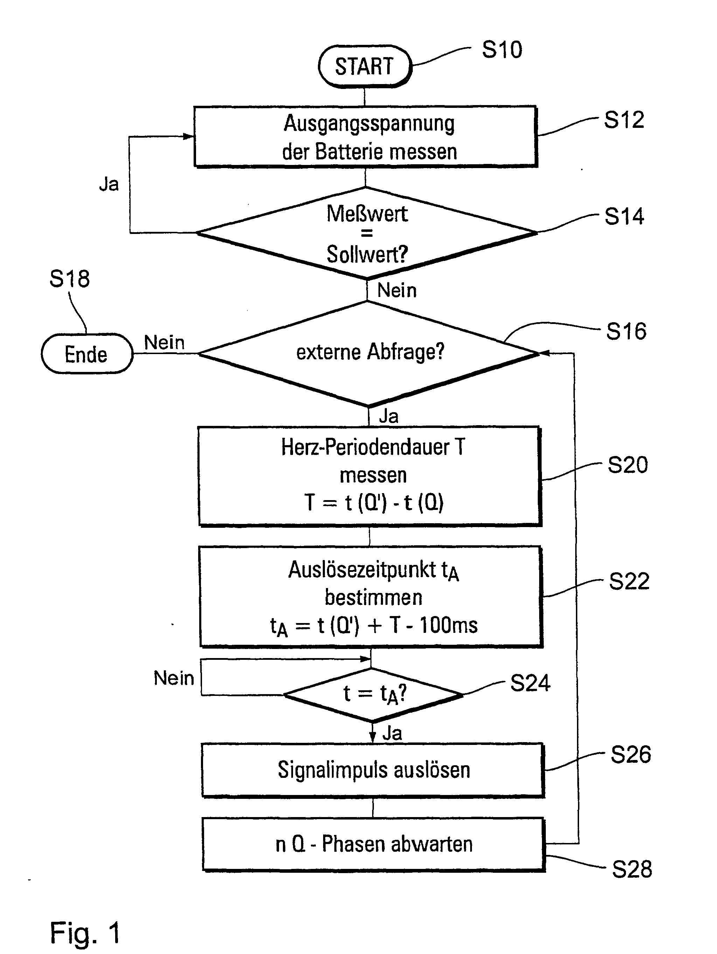

Figur 1 zeigt in einem Flussdiagramm ein Ausführungsbeispiel des erfindungsgemäßen Signalisierungsverfahrens.

Das Verfahren wird mit einem Schritt S10 gestartet. In einem anschliessenden Schritt S12 wird als Zustandsgröße die Ausgangsspannung einer Batterie gemessen. Die Batterie dient zur Energieversorgung einer implantierbaren Herzstimulationsvorrichtung. In einem anschließenden Schritt S14 wird die gemessene Ausgangsspannung mit einem Sollwert verglichen. Es versteht sich, dass anstelle eines Sollwertes auch ein Sollwertintervall zum Vergleich herangezogen werden kann.

Stimmt der Messwert mit dem Sollwert überein, wird anschließend erneut der Schritt S12 ausgeführt. Die von den Schritten S12 und S14 gebildete Schleife dient also der Überwachung der Batteriespannung. Vor der erneuten Ausführung des Schrittes S12 nach festgestellter Übereinstimmung zwischen Messwert und Sollwert kann auch eine Warteschleife vorbestimmter Dauer durchlaufen werden.

Wird im Schritt S14 festgestellt, dass die gemessene Ausgangsspannung nicht mit dem Sollwert bzw. Sollwertintervall übereinstimmt, so wird in einem nachfolgenden Schritt S16 geprüft, ob ein magnetisches Auslösesignal vorliegt. In einer alternativen Ausführungsform wird dieser Verfahrensschritt S 16 zusätzlich zwischen den Schritten S10 und S12 ausgeführt. Das heißt, dass in dieser Ausführungsform eine Messung der Ausgangsspannung erst auf das magnetische Auslösesignal hin durchgeführt wird.

Ist kein magnetisches Auslösesignal nachweisbar, wird das Verfahren mit Schritt S18 beendet. Alternativ kann auch eine Warteschleife längerer Dauer vorgesehen sein, nach deren Ende erneut das Vorhandensein des magnetischen Auslösesignals geprüft wird.

Das magnetische Auslösesignal wird in bekannter Weise durch Auflegen eines externen Programmier- und Prüfgerätes für die Herzstimulationsvorrichtung auf die Brust des Patienten erzeugt. Es versteht sich jedoch, dass die Implantation der Herzstimulationsvorrichtung nicht Voraussetzung für die Durchführung des Verfahrens ist. Das Auslösesignal kann ebenso nachgewiesen werden, wenn die Herzstimulationsvorrichtung nicht implantiert ist.

Wird ein magnetisches Auslösesignal nachgewiesen, so wird in einem Schritt S20 die aktuelle Herzperiodendauer als Zeitdifferenz

$$T = t(Q') - t(Q)$$

zwischen zwei Zeitpunkten t (Q') und t(Q) zweier aufeinanderfolgender Herzperioden bestimmt, an denen jeweils das Spannungsminimum der Q-Zacke im Elektrokardiogramm durchlaufen wird.

Es versteht sich, dass anstelle der Q-Zacke des EKGs auch eine andere Phase der Erregung des Myokards im Herzzyklus zur Messung der Periodendauer herangezogen werden kann. Das Elektrokardiogramm wird in bekannter Weise mit Hilfe der Stimulationselektroden oder eigens hierfür vorgesehener Messelektroden aufgenommen.

In einem anschließenden Schritt S22 wird der Zeitpunkt $t_A$ vorausberechnet, an dem ein Signalimpuls erzeugt werden soll. Hierbei wird auf eine vorbestimmte Zeitkonstante $\Delta t$, zurückgegriffen. Im vorliegenden Beispiel beträgt $\Delta t$ 100ms. Der Auslösezeitpunkt wird im Schritt S22 nach der Formel

$$t_A = t(Q') + T - \Delta t$$

bestimmt.

Dabei beschreibt die Summe t (Q') + T den hypothetischen Zeitpunkt des nächsten Eintretens der Q-Zacke für den Fall, daß die Herzperiode unverändert bliebe. Selbstverständlich ist der genaue Zeitpunkt des Eintretens der nächsten Q-Zacke grundsätzlich nicht vorauszubestimmten. Die Annahme einer identischen Periodendauer zweier aufeinanderfolgender Herzperioden stellt jedoch eine gute und für die externe Erkennbarkeit des Signalimpulses ausreichend genaue Annahme dar. Wichtig ist, dass mit Hilfe dieser Summe im Schritt S22 der Zeitpunkt des Eintretens einer bestimmten Herzphase vorausberechnet wird.

Anschließend wird mit einem Schritt S24 das Eintreten des Zeitpunktes $t_A$ abgewartet. Im Zeitpunkt $t_A$ wird mit einem Schritt S26 ein Signalimpuls ausgelöst.

Anschließend wird mit einem Schritt S28 eine vorbestimmte Anzahl n von Herzperioden abgewartet. Dabei werden die auftretenden Q-Phasen bis n hochgezählt, bevor zurück zu Schritt S16 gesprungen wird, um erneut zu prüfen, ob weiterhin ein magnetisches Auslösesignal vorliegt. Ist dies der Fall, werden anschließend wiederum die Schritte S20 bis S28 ausgeführt. Liegt jedoch kein magnetisches Auslösesignal mehr vor, wird das Verfahren mit Schritt S18 beendet.

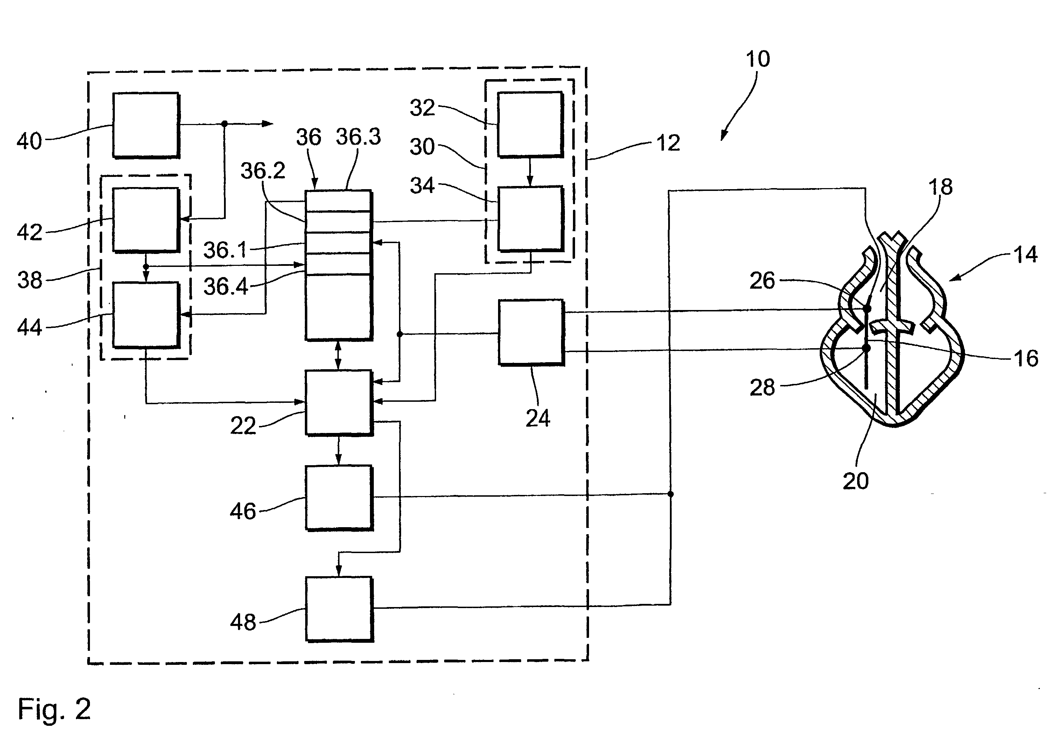

Figur 2 zeigt ein vereinfachtes Blockschaltbild eines Ausführungsbeispiels der erfindungsgemäßen Herzstimulationsvorrichtung. Hierbei handelt es sich um einen integrierten Kardioverter/Defibrillator (ICD) 10, der zusätzlich zur Durchführung einer Zweikammer-Schrittmachertherapie ausgebildet ist. Dargestellt ist der ICD 10 implantierten Zustand.

Der ICD 10 weist einen implantierbaren Impulsgenerator 12 und eine mit diesem verbundene und in ein Herz 14 implantierte Stimulationselektrode 16 auf. Die Stimulationselektrode 16 ist zur Abgabe von Stimulationsimpulsen im rechten Vorhof 18 und im rechten Ventrikel 20 ausgebildet.

Der Impulsgenerator 12 weist eine zentrale Steuereinheit 22 auf. Sie steuert, koordiniert und überwacht die Funktionsmerkmale des Impulsgenerators 12. Sie empfängt und verarbeitet hierfür die Signale und Daten verschiedener Messeinheiten.

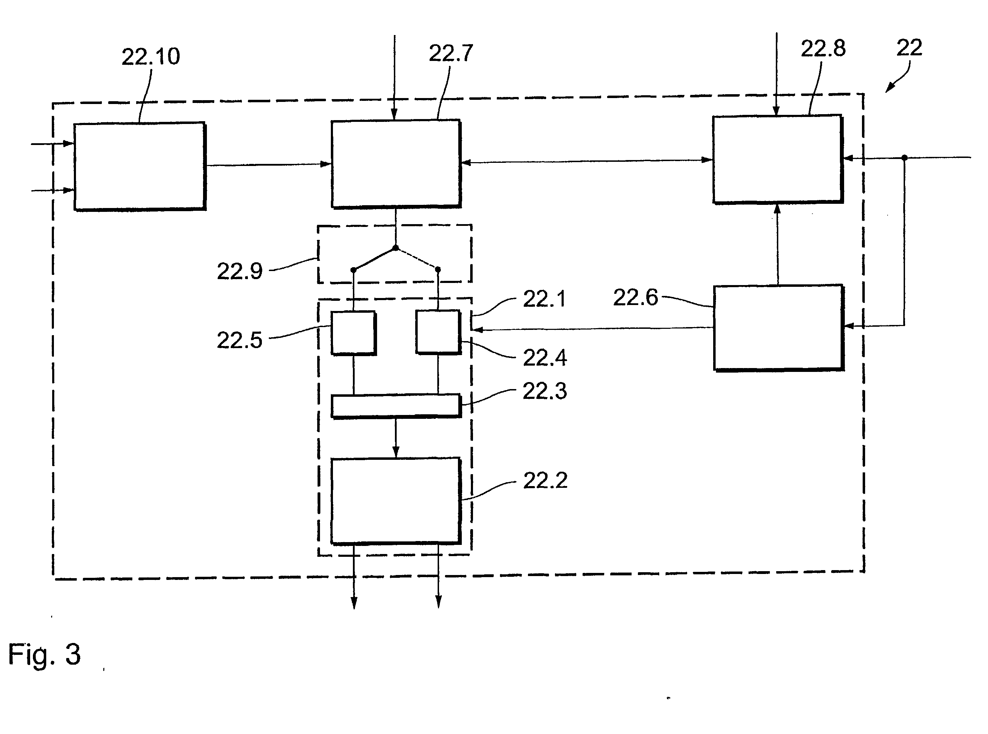

So ist die Steuereinheit 22 zum einen mit einer Elektrokardiographie(EKG)-Einheit 24 verbunden. Diese nimmt mit Hilfe zweier an der Stimulationselektrode 16 ausgebildeter Messelektroden 26 und 28 ein intrakardiales Elektrogramm (IEGM) auf. Das Messsignal wird zur Weiterverarbeitung an die Steuereinheit 22 geleitet. Nähere Einzelheiten hierzu werden anhand der Figur 3 beschrieben. Zugleich kann das Messsignal von der Steuereinheit kontrolliert in einem Bereich 36.1 eines Speichers 36 abgelegt werden, um später bei Bedarf zur Langzeitauswertung abgerufen zu werden.

Zum anderen empfängt die Steuereinheit 22 Signale und Daten von der Telemetrieeinheit 30. Die Telemetrieeinheit 30 weist einen Empfänger 32 für magnetische Signale eines (nicht dargestellten) externen Abfrage-, Programmier- und Überwachungsgerätes auf. Eine Vergleicherschaltung 34 ist mit dem Empfänger 32 verbunden und dient zur Identifizierung der detektierten Magnetsignale. Hierzu greift die Vergleicherschaltung 34 auf in einem Speicherbereich 36.2 abgelegte Sollwertund Zuordnungstabellen zu. Nach Identifizierung des empfangenen Signals signalisiert die Vergleicherschaltung 34 der Steuereinheit 22, welches Funktionsmerkmal des Impulsgenerators 12 von extern abgefragt wird.

Schließlich empfängt die Steuereinheit 22 Signale von einem EOL-Indikator 38 (EOL = end of life), der den Energieinhalt einer Batterie 40 überwacht. Der EOL-Indikator 38 weist einen mit dem Ausgang der Batterie 40 verbundenen Spannungsmesser 42 auf. Die Batterie 40 dient zur Energieversorgung des Impulsgenerators 12. Eine zweite Vergleicherschaltung 44 dient zum Vergleich der gemessenen Ausgangsspannung der Batterie 40 mit einem in einem Speicherbereich 36.3 abgelegten Sollwertintervall. Sinkt die Ausgangsspannung unter einen durch das Sollwertintervall vorgegebenen unteren Schwellwert, erzeugt der EOL-Indikator 38 ein EOL-Signal, das an die Steuereinheit übermittelt wird.

Es versteht sich, dass im Speicher in bekannter Weise auch mehrere Wertintervalle für die Ausgangsspannung der Batterie 40 abgelegt sein können und die zweite Vergleicherschaltung 44 dementsprechend differenzierte Signale erzeugen kann. Ein erstes Wertintervall entspricht dabei beispielsweise der vollen Funktionsfähigkeit der Batterie, ein zweites entspricht einer Ausgangsspannung, bei der die baldige Auswechslung der Batterie (Elective Replacement Interval = ERI) empfohlen ist und ein drittes Wertintervall entspricht einer Ausgangsspannung, bei der die Aufrechterhaltung der Funktion des Impulsgenerators gefährdet ist.

Mit der Steuereinheit 22 verbunden sind weiterhin eine Schrittmachereinheit 46 und eine Kardioversionseinheit 48. Diese übernehmen in Abhängigkeit von den von der Steuereinheit 22 her emfangenen Steuersignalen in bekannter Weise die Erzeugung und Abgabe von Herzstimulationsimpulsen bzw. Kardiversions- oder Defibrillationsimpulsen.

Figur 3 zeigt nähere Einzelheiten der für die Darstellung der Erfindung wesentlichen Strukturmerkmale der Steuereinheit 22 in einem vereinfachten Blockdiagramm.

Die Steuereinheit 22 weist eine Signalgebereinheit 22.1 mit einem Signalgeber 22.2 auf. Der Signalgeber ist zur Abgabe von Steuerimpulsen an die Schrittmachereinheit 46 oder die Kardioversionseinheit 48 ausgebildet. Ein vom Signalgeber erzeugter Steuerimpuls veranlasst die Schrittmachereinheit 46 bzw. die Kardioversionseinheit 48 zur unmittelbaren Erzeugung und Abgabe eines Herzstimulationsimpulses. Der Empfänger des vom Signalgeber erzeugten Steuerimpulses, also entweder die Schrittmachereinheit 46 oder die Kardioversionseinheit 48, wird mit Hilfe eines dem Signalgeber 22.2 Adressierers 22.3 bestimmt.

Dem Adressierer parallel vorgeschaltet sind eine Triggereinheit 22.4 sowie eine Zeitabstimmeinheit 22.5. Beide sind zur Bestimmung des Zeitpunktes der Abgabe eines Steuerimpulses durch den Signalgeber 22.2 ausgebildet. Die Triggereinheit 22.4 erzeugt die Steuersignale nach einem durch eine Therapiesteuereinheit 22.6 vorgegebenen Programm, wie es von herkömmlichen ICDs mit Schrittmacherfunktionen bekannt ist. Die Zeitabstimmeinheit 22.5 ist Bestimmung des Zeitpunktes der Abgabe von Steuerimpulsen für die Erzeugung zeitversetzter Herzstimulationsimpulse ausgebildet. Hierzu sind in der Zeitabstimmeinheit 22.5 Verfahrensschritte implementiert, die anhand von Figur 1 beschrieben wurden.

Zu beachten ist jedoch, daß die Zeitabstimmeinheit nicht zur Abgabe von Signalimpulsen, sondern von Steuersignalen an den Signalgeber 22.2 ausgebildet ist. Sie bestimmt den Zeitpunkt der Abgabe eines Herzstimulationsimpulses und erzeugt ein dementsprechendes Steuersignal. Diesem Steuersignal entnimmt der Adressierer 22.3, daß die Schrittmachereinheit 46 vom Signalgeber 22.2 anzusteuern ist. Dieser wiederum entnimmt dem Steuersignal der Zeitabstimmeinheit 22.5 den Zeitpunkt, an dem der Herzstimulationsimpuls abzugeben ist und veranlasst dies durch Abgabe eines entsprechenden Impulsauslösesignals an die Schrittmachereinheit 46.

Die Zeitabstimmeinheit 22.5 greift auf das Ausgangssignal einer Periodendauer-Messeinheit 22.6 zu, die im folgenden kurz als T-Messeinheit bezeichnet wird. Das Ausgangssignal der T-Messeinheit 22.6 gibt den Zeitabstand zwischen der letzten und vorletzten Q-Zacke sowie den Zeitpunkt des Eintretens der letzten Q-Zacke an. Das Ausgangssignal der T-Einheit 22.6 enthält also zeitnahe Informationen über die Periodendauer und die Phasenlage der Herztätigkeit.

Die Zeitabstimmeinheit 22.5 bestimmt den Zeitpunkt der Abgabe eines Stimulationsimpulses durch Addition der gemessenen Periodendauer zum Zeitpunkt des Eintretens der letzten Q-Zacke und anschließende Subtraktion eines vorbestimmten Zeitversatzes von beispielsweise 100ms. Der Wert des Zeitversatzes kann durch Programmierung von extern vorgegeben werden und ist im Speicher 36 abgelegt.

Eine Therapiesteuereinheit 22.7 kontrolliert mit Hilfe einer Herzüberwachungseinheit 22.8 den Zustand des Herzens und bestimmt mit Hilfe bekannter Diagnoseverfahren, welche Therapieform im jeweiligen Augenblick anzuwenden ist. Die Therapiesteuerung steuert den Zugriff auf die Triggereinheit oder die Zeitabstimmeinheit nach Art einer Umschaltvorrichtung 22.9. Die Zeitabstimmeinheit wird von der Therapiesteuereinheit nur angesteuert, wenn eine Signalisierung nach extern vorgenommen werden soll. Zur Durchführung der üblichen Therapieformen wird die Triggereinheit 22.4 mit Hilfe bekannter Therapiesteuerungsverfahren betrieben.

Die Therapiesteuereinheit 22.6 ist eingangsseitig zusätzlich mit einer Geräteüberwachungseinheit 22.10 verbunden. Die Geräteüberwachungseinheit empfängt ihrerseits die Ausgangssignale der ersten und zweiten Vergleicherschaltungen 34 und 44. Zeigt die zweite Vergleicherschaltung 44 an, dass die Batteriespannung unterhalb des Sollwertbereiches liegt, und zeigt erste Vergleicherschaltung 34 der Telemetrieeinheit 30 zugleich an, dass von extern der Zustand der Batterie abgefragt wird, leitet die Geräteüberwachungsschaltung 22.10 das EOL- oder ERI-Signal Vergleicherschaltung 44 an die Therapiesteuereinheit 22.7 weiter.

Die Therapiesteuereinheit 22.7 fragt auf den Empfang eines EOL- oder ERI-Signals hin die Herzüberwachungseinheit 22.8 auf das Vorliegen eines pathologischen Zustands des Herzens ab. Liegt ein solcher vor, wird eine entsprechende Therapie veranlaßt. Arbeit das Herz - stimuliert oder im natürlichen Rhythmus - normal, wird mit Hilfe der Umschaltvorrichtung 22.9 die Zeitabstimmeinheit aktiviert.

Es versteht sich, dass in gleicher Weise andere interne Zustände der Herzstimulationsvorrichtung signalisiert werden können.

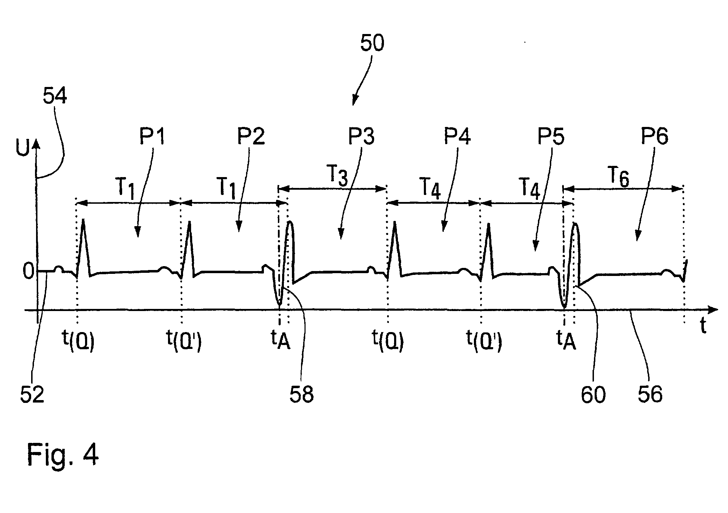

Figur 4 zeigt in schematischer Darstellung ein während einer Signalisierung durch eine erfindungsgemäße Herzstimulationsvorrichtung extern aufgenommenes Elektrokardiogramm 50. An einer Abszisse 52 ist die Zeit t in nicht näher angegebenen Einheiten und an einer Ordinate 54 ist eine Messspannung U, ebenfalls in unbestimmten Einheiten aufgetragen.

Eine resultierende EKG-Kurve 52 zeigt den bekannten Verlauf über die Dauer von fünf Herzperioden P1 bis P5. Die EKG-Kurve 52 gibt das EKG einer natürlichen Herztätigkeit wieder. Die Periodendauer der Perioden P1, P3 und P4 wird durch Pfeile T1, T3, T4 und T6 gekennzeichnet, deren Anfangs- und Endpunkte jeweils mit dem Eintreten einer Q-Zacke in der EKG-Kurve 52 übereinstimmen.

Die Periodendauern der Perioden P1 bis P5 sind aufgrund der natürlichen Schwankungen der Herztätigkeit nicht gleich. Im vorliegenden Fall ist T4 etwas kürzer als T1, T3 und T6. Mit gleichen Bezugszeichen gekennzeichnete Pfeile bezeichnen Zeitspannen gleicher Länge.

Durch vertikale, gestrichelte Linien sind die Anfangs- und Endzeitpunkte der Perioden P1 bis P5 gekennzeichnet. Der Anfangszeitpunkt der Periode P1 ist unterhalb der Abszisse 56 mit t(Q) bezeichnet. Dies ist der Zeitpunkt des Eintretens der Q-Zacke der Herzperiode P1. Der Zeitpunkt des Eintretens der darauffolgenden Q-Zacke ist mit t(Q') gekennzeichnet.

Bei der Durchführung des erfindungsgemäßen Verfahrens mit Hilfe einer erfindungsgemäßen Herzstimulationsvorrichtung wird entsprechend dem Schritt S20 in Figur 1 durch die T-Messeinheit 22.6 aus Figur 3 zunächst die Zeitspanne T1 bestimmt. Im Verlauf der Anfangsphase der Herzperiode P2 bestimmt die Zeitabstimmeinheit 22.5 den Auslösezeitpunkt $t_A$ für einen Signalimpuls. Dieser ist in Figur 4 zusätzlich durch eine strichpunktierte vertikale Linie gekennzeichnet. Zu erkennen ist ein Versatz dieser strichpunktierten Linie $t_A$ relativ zum rechten Ende des sich über die Herzperiode P2 erstreckenden Pfeils T1. Dieser zeigt also an, wann die Zeitspanne T1, also die Dauer der Herzperiode P1, nach Beginn der Periode P2 erneut abgelaufen wäre.

In der EKG-Kurve 52 ist zum Zeitpunkt $t_A$ einsetzend ein starker Ausschlag 58 zu negativen Spannungswerten hin zu erkennen. Dieser Ausschlag ist das im EKG sichtbare Anzeichen eines von der Schrittmachereinheit 46 abgegebenen Herzstimulationsimpulses. Durch seinen Zeitversatz relativ zum Zeitpunkt t(Q') + T1 ist dieser Herzstimulationsimpuls von einem regulären, therapeutischen Herzstimulationsimpuls unterscheidbar und als Signalimpuls identifizierbar. Dieser zeigt also an, daß die Batteriespannung der Herzstimulationsvorrichtung 10 einen Sollwert unterschritten hat.

Da die Identifizierung nur eines Signalimpulses nicht immereindeutig ist, wiederholt sich das Geschehen nach zwei Herzperioden P4 und P5 in der Herzperiode P6. Das Abwarten von zwei Perioden ohne Signalisierung entspricht dem Schritt S28 des Vefahrens nach Figur 1, wobei die Anzahl zwei der abzuwartenden Perioden nach Programmierung von extern im Speicher 36 (Figur 2) abgelegt ist.

Die Zeitabstimmeinheit 22.5 (Figur 3) greift zur Bestimmung des Auslösezeitpunktes $t_A$ in der Periode P6 auf die Periodendauer T4 und den Zeitpunkt t(Q') des Beginns der Periode P5 zurück, die von der T-Messeinheit 22.6 bestimmt werden. Die EKG-Kurve 52 zeigt entsprechend einen zweiten negativen Aussschlag 60.

Dieses zeitliche Muster wird in der EKG-Kurve 52 auch für spätere, hier nicht dargestellte Zeiten wiederholt zu erkennen sein, solange die Telemetrieeinheit die externe Empfangsbereitschaft für das Signal anzeigt und die Herztätigkeit normal ist.

**Patentansprüche**

1. Verfahren zur Signalisierung eines internen Zustandes einer implantierbaren Herzstimulationsvorrichtung, der durch einen Wert einer Zustandsgröße definiert ist, umfassend folgende Schritte:

   - Aufnahme eines die Zustandsgröße repräsentierenden Messwerts (S12),
   - Ermitteln des Vorliegens des Zustandes durch Vergleich des Messwerts mit einem Vergleichswert (S14),
   - Abgabe eines Signalimpulses mit einem vorbestimmten, messbaren Impulsparameter, wobei der Impulsparameter dem Zustand eindeutig zugeordnet ist, falls der Messwert dem Vergleichswert innerhalb vorgegebener Grenzen entspricht (S26),

   **gekennzeichnet durch** die Schritte:

   - Messen der Zeitdifferenz gleicher, vorbestimmter Phasen zweier aufeinanderfolgender, periodisch wiederkehrender Ereignisabfolgen vor Abgabe des Signalimpulses (S20), sowie anschließend
   - Abgabe des Signalimpulses um eine vorbestimmbare und dem Zustand eindeutig zugeordnete Zeitspanne vor oder nach dem nächsten Ablauf der gemessenen Zeitdifferenz (S22, S24, S26).

2. Verfahren nach Anspruch 1, **dadurch gekennzeichnet, daß** es gestartet wird, sobald in der Vorrichtung ein Auslösesignal vorliegt.

3. Verfahren nach Anspruch 2, **dadurch gekennzeichnet, daß** es erneut durchgeführt wird, wenn nach der Durchführung das Auslösesignal weiter vorliegt.

4. Verfahren nach Anspruch 2 oder 3, **dadurch gekennzeichnet, daß** das Auslösesignal erzeugt wird, solange in der Vorrichtung ein Magnetfeld mit einem vorbestimmten Magnetfeldparameter detektiert wird.

5. Verfahren nach einem der vorstehenden Ansprüche, **dadurch gekennzeichnet, daß** im Anschluß an die Abgabe des zeitversetzten Signalimpulses für eine vorbestimmbare Anzahl nachfolgender Ereignisabfolgen kein zeitversetzter Signalimpuls erzeugt wird (S28).

6. Verfahren nach einem der vorstehenden Ansprüche, **dadurch gekennzeichnet** durch das Messen einer von der Phase des Herzzyklus abhängige physikalische Größe.

7. Verfahren nach Anspruch 6, **gekennzeichnet durch** das ein Aufnehmen eines Elektrodkardiogramms.

8. Verfahren nach einem der vorstehenden Ansprüche, **dadurch gekennzeichnet, daß** als Messwert eine am Ausgang eines in der Vorrichtung angeordneten Energiespeichers anliegende elektrische Spannung aufgenommen wird (S12).

9. Verfahren nach einem der vorstehenden Ansprüche, **dadurch gekennzeichnet, daß** als Signalimpuls ein elektrischer Stromimpuls abgegeben wird.

10. Herzstimulationsvorrichtung mit

   - einem Impulsgenerator (12), der für die Erzeugung von zur Herzstimulation geeigneten Impulsen ausgebildet ist,
   - einer mit dem Impulsgenerator (12) verbundenen und zur Abgabe der Impulse ausgebildeten Stimulationselektrode (16),
   - einem Signalgeber (22.2), der mit dem Impulsgenerator (12) verbunden und zur Erzeugung und Abgabe von Impulsauslösesignalen an definierten Zeitpunkten ($t_A$) ausgebildet ist,
   - einem Herzphasenmessfühler (24, 26, 28), der zur Aufnahme eines von der aktuellen Phase des Herzzyklus abhängigen ersten Messwertes ausgebildet ist, und mit
   - Zeitmessmitteln (22.9), die mit dem Herzphasenmessfühler (24, 26, 28) verbunden und zur Bestimmung einer Zyklusdauer zwischen dem Eintreten einer vorbestimmten Phase (Q) eines definierten ersten Herzzyklus (P1, P4) und dem Eintreten der gleichen Phase (Q') des unmittelbar nachfolgenden, zweiten Herzzyklus (P2, P5) ausgebildet sind,

   wobei
   eingangsseitig mit den Zeitmessmitteln (22.9) sowie ausgangsseitig mit dem Signalgeber (22.2) verbundene Zeitabstimmmittel (22.5) vorgesehen sind, die zum Vorbestimmen der Zeitspanne zwischen dem Eintreten der vorbestimmten Phase (Q') des zweiten Herzzyklus und der nachfolgenden Erzeugung eines Impulsauslösesignals ausgebildet sind, und

ein mit den Zeitabstimmmitteln (22.5) verbundener Zustandsmessfühler (42), der zur Aufnahme eines Zustandsmesswertes ausgebildet ist, welcher vom aktuellen Wert einer Zustandsgröße der Vorrichtung abhängt.

11. Herzstimulationsvorrichtung mit

- einem Impulsgenerator (12), der für die Erzeugung von zur Herzstimulation geeigneten Impulsen ausgebildet ist,
- einer mit dem Impulsgenerator (12) verbundenen und zur Abgabe der Impulse ausgebildeten Stimulationselektrode (16),
- einem Signalgeber (22.2), der mit dem Impulsgenerator (12) verbunden und zur Erzeugung und Abgabe von Impulsauslösesignalen an definierten Zeitpunkten ($t_A$) ausgebildet ist,
- einem Herzphasenmessfühler (24, 26, 28), der zur Aufnahme eines von der aktuellen Phase des Herzzyklus abhängigen ersten Messwertes ausgebildet ist, und mit
- Zeitmessmitteln (22.9), die mit dem Herzphasenmessfühler (24, 26, 28) verbunden und zur Bestimmung einer Zyklusdauer zwischen dem Eintreten einer vorbestimmten Phase (Q) eines definierten ersten Herzzyklus (P1, P4) und dem Eintreten der gleichen Phase (Q') des unmittelbar nachfolgenden, zweiten Herzzyklus (P2, P5) ausgebildet sind,

wobei
eingangsseitig mit den Zeitmessmitteln (22.9) sowie ausgangsseitig mit dem Signalgeber (22.2) verbundene Zeitabstimmmittel (22.5) vorgesehen sind, die zum Vorbestimmen der Zeitspanne zwischen dem Eintreten der vorbestimmten Phase (Q') des zweiten Herzzyklus und der nachfolgenden Erzeugung eines Impulsauslösesignals ausgebildet sind und
ein mit den Zeitabstimmmitteln (22.5) verbundener Zustandsmessfühler (42), der zur Aufnahme eines Zustandsmesswertes ausgebildet ist, welcher vom aktuellen Wert einer Zustandsgröße der Vorrichtung abhängt.

12. Vorrichtung nach Anspruch 11, **dadurch gekennzeichnet, daß** der Zustandsmessfühler (42) so ausgebildet ist, daß der Zustandsmesswert vom Energieinhalt eines Energiespeichers (40) der Vorrichtung (10) abhängt.

13. Vorrichtung nach Anspruch 11 oder 12, **gekennzeichnet durch** einen mit den Zeitabstimmmitteln (22.5) verbundenen Vergleichswertspeicher (36.3) zur Aufnahme eines Vergleichswertes für den Zustandsmesswert.

14. Vorrichtung nach Anspruch 13, **gekennzeichnet durch** erste Vergleichsmittel (44), die eingangsseitig mit dem Zustandsmessfühler (42) und dem Vergleichswertspeicher (36.3) sowie ausgangsseitig mit den Zeitabstimmmitteln (22.5) verbunden sind und die zum Vergleich des Zustandsmesswertes mit dem Vergleichswert sowie zur Erzeugung und Abgabe eines vom Vergleichsergebnis abhängigen Zustandssignals ausgebildet sind.

15. Vorrichtung nach Anspruch 14, **gekennzeichnet durch** eine den Zeitabstimmmitteln (22.5) vorgeschaltete und eingangsseitig mit den ersten Vergleichsmitteln (44) verbundene Überwachungseinheit (22.10), die zum Vergleich des Zustandssignals mit einem vorgespeicherten Zustandssollwertintervall und zur Erzeugung und Abgabe eines Wartungssignals ausgebildet ist für den Fall, daß das Zustandssignal nicht im Zustandssollwertintervall enthalten ist.

16. Vorrichtung nach einem der Ansprüche 10 bis 15, **dadurch gekennzeichnet, daß** ein mit den Zeitabstimmmitteln (22.5) verbundener Aktivierungsmessfühler (32) vorgesehen ist, der zur Aufnahme eines Aktivierungsmesswertes einer Aktivierungsmessgrösse ausgebildet ist.

17. Vorrichtung nach Anspruch 16, **dadurch gekennzeichnet, daß** der Aktivierungsmessfühler (32) ein Magnetfeldsensor ist.

18. Vorrichtung nach Anspruch 16 oder 17, **gekennzeichnet durch** einen Musterwertspeicher (36.2) zur Aufnahme eines Musterwertes für den Aktivierungsmesswert.

19. Vorrichtung nach Anspruch 18, **gekennzeichnet durch** zweite Vergleichsmittel (34), die eingangsseitig mit dem Aktivierungsmessfühler (32) und dem Musterwertspeicher (36.2) sowie ausgangsseitig mit den Zeitabstimmmitteln (22.5) verbunden sind und die zum Vergleich des Aktivierungsmesswertes mit dem Musterwert sowie im Falle der Übereinstimmung zur Erzeugung und Abgabe eines Auslösesignals ausgebildet sind.

20. Vorrichtung nach Anspruch 14, 15 und 19, **dadurch gekennzeichnet, daß** die Überwachungseinheit (22.10) eingangsseitig zusätzlich mit den zweiten Vergleichsmitteln (34) verbunden und zusätzlich so gestaltet ist, daß das Wartungssignal nur erzeugbar ist, wenn ein Auslösesignal vorliegt.

21. Vorrichtung nach einem der Ansprüche 10 bis 20, **dadurch gekennzeichnet, daß** der Herzphasenmessfühler (24, 26, 28) zur Aufnahme eines von der elektrischen Polarisation des Myokards abhängigen Messsignals ausgebildet ist.

22. Vorrichtung nach einem der Ansprüche 10 bis 21, **dadurch gekennzeichnet, daß** die Zeitmessmittel (22.9) zur Bestimmung des QQ-Zeitintervalls aufeinanderfolgender Herzzyklen ausgebildet sind.

23. Vorrichtung nach einem der Ansprüche 10 bis 22, **dadurch gekennzeichnet, daß** die Zeitabstimmittel (22.5) zur Erzeugung des nachfolgenden Impulsauslösesignals nicht früher als 300 Millisekunden vor dem Ablauf der bestimmten Zyklusdauer nach dem Eintreten der vorbestimmten Phase (Q') des zweiten Herzzyklus (P2, P5) ausgebildet sind.

24. Vorrichtung nach Anspruch 23, **dadurch gekennzeichnet, daß** die Zeitabstimmittel (22.5) zur Erzeugung des nachfolgenden Impulsauslösesignals nicht früher als 200 Millisekunden vor dem Ablauf der bestimmten Zyklusdauer nach dem Eintreten der vorbestimmten Phase (Q') des zweiten Herzzyklus ausgebildet sind.

25. Vorrichtung nach einem der Ansprüche 10 bis 24, **dadurch gekennzeichnet, daß** die Zeitabstimmittel (22.5) zur Erzeugung des nachfolgenden Impulsauslösesignals nicht später als 50 Millisekunden vor dem Ablauf der bestimmten Zyklusdauer nach dem Eintreten der vorbestimmten Phase des zweiten Herzzyklus (Q') ausgebildet sind.

26. Vorrichtung nach einem Anspruch 25, **dadurch gekennzeichnet, daß** die Zeitabstimmittel (22.5) zur Erzeugung des nachfolgenden Impulsauslösesignals nicht später als 100 Millisekunden vor dem Ablauf der bestimmten Zyklusdauer nach dem Eintreten der vorbestimmten Phase (Q') des zweiten Herzzyklus ausgebildet sind.

27. Vorrichtung nach einem der Ansprüche 10 bis 26, **gekennzeichnet durch** mit den Zeitabstimmitteln (22.5) verbundene Schrittmachersteuermittel (22.7), die zur wahlweisen Erzeugung eines Aktivierungs- oder eines Deaktivierungssignals zur Aktivierung bzw. Deaktivierung des Betriebs der Zeitabstimmittel (22.5) ausgebildet sind.

28. Vorrichtung nach Anspruch 27, **dadurch gekennzeichnet, daß** die Schrittmachersteuermittel (22.7) eingangsseitig mit dem Herzphasenmessfühler (24, 26, 28) verbunden und zur Aktivierung oder Deaktivierung des Betriebs der Zeitabstimmittel (22.5) für eine vorbestimmbare Anzahl von Herzzyklen ausgebildet sind.

29. Vorrichtung nach Anspruch 28, **dadurch gekennzeichnet, daß** die Schrittmachersteuermittel (22.7) zur Aktivierung des Betriebs der Zeitabstimmittel (22.5) für die Dauer eines Herzzyklus (P2, P5) und zur unmittelbar anschließenden Deaktivierung des Betriebs der Zeitabstimmittel (22.5) ausgebildet sind.

30. Vorrichtung nach Anspruch 29, **dadurch gekennzeichnet, daß** die Schrittmachersteuermittel (22.7) zur unmittelbar anschließenden Deaktivierung des Betriebs der Zeitabstimmittel (22.5) für die Dauer von mindestens zwei Herzzyklen (P3, P4) ausgebildet sind.

31. Vorrichtung nach Anspruch 29 oder 30, **dadurch gekennzeichnet, daß** die Schrittmachersteuermittel (22.7) zur unmittelbar anschließenden Deaktivierung des Betriebs der Zeitabstimmittel (22.5) für die Dauer von höchstens 15 Herzzyklen ausgebildet sind.

32. Vorrichtung nach einem der Ansprüche 29 bis 31, **dadurch gekennzeichnet, daß** die Schrittmachersteuermittel (22.7) zur unmittelbar anschließenden Deaktivierung des Betriebs der Zeitabstimmittel (22.5) für die Dauer von 5 Herzzyklen ausgebildet sind.

33. Vorrichtung nach einem der Ansprüche 10 bis 32, **dadurch gekennzeichnet**, den Schrittmachersteuermitteln (22.7) eine mit den Zeitmessmitteln (22.9) verbundene Herzüberwachungseinheit (22.8) vorgeschaltet ist, die zum Vergleich der jeweils gemessenen Zyklusdauer (T1, T3, T4, T6) mit einem in einem Zyklussollwertspeicher enthaltenen Zyklusdauer-Sollwertintervall ausgebildet ist.

34. Vorrichtung nach Anspruch 33, **dadurch gekennzeichnet, daß** die Herzüberwachungseinheit (22.8) für den Fall des Vorliegens einer Abweichung der gemessenen Zyklusdauer (T1, T3, T4, T6) vom Zyklusdauer-Sollwertintervall zur Erzeugung eines vom Wert der Abweichung abhängigen Therapiewarnsignals ausgebildet ist.

35. Vorrichtung nach Anspruch 33 oder 34, **dadurch gekennzeichnet, daß** die Schrittmachersteuermittel (22.7) zur Erzeugung und Übermittlung eines Deaktivierungssignals an die Zeitabstimmittel (22.5) bei Empfang eines Therapiewarnsignals ausgebildet sind.

36. Vorrichtung nach einem der Ansprüche 33 bis 35, **dadurch gekennzeichnet, daß** die Schrittmachersteuermittel (22.7) zur Blockierung der Erzeugung eines Aktivierungssignals für eine definierte Zeitspanne bei Empfang eines Therapiewarnsignals ausgebildet sind.

37. Vorrichtung nach einem der Ansprüche 10 bis 36 **gekennzeichnet durch** eine eingangsseitig mit dem Herzphasenmessfühler (24, 26, 28) und ausgangsseitig mit dem Signalgeber (22.2) verbundene Triggereinheit (22.4), die zur Erzeugung und Abgabe von Triggersignalen an den Signalgeber (22.2) zu definierten Zeitpunkten im Rahmen einer Schrittmachertherapie ausgebildet ist, wobei die Triggersignale den Signalgeber (22.2) zur Erzeugung und Abgabe von Impulsauslösesignalen veranlassen.

**38.** Vorrichtung nach Anspruch 37, **dadurch gekennzeichnet, daß** die Schrittmachersteuermittel (22.7) zusätzlich mit der Triggereinheit (22.4) verbunden und zur Erzeugung und Abgabe eines Triggeraktivierungs- oder -deaktivierungssignals an die Triggereinheit (22.4) ausgebildet sind, wobei bei Abgabe eines Triggeraktivierungssignals an die Triggereinheit (22.4) auch die Abgabe eines Deaktivierungssignals an die Zeitabstimmmittel (22.5) und, vice versa, bei Abgabe eines Aktivierungssignals an die Zeitabstimmmitel (22.5) auch die Abgabe eines Triggerdeaktivierungssignals an die Triggereinheit (22.4) erfolgt.

**39.** Vorrichtung nach Anspruch 38, **dadurch gekennzeichnet, daß** die Schrittmachersteuermittel (22.7) zur Erzeugung eines Triggeraktivierungssignals bei Vorliegen eines Therapiewarnsignals ausgebildet sind.

**40.** Vorrichtung nach einem der Ansprüche 10 bis 39, **gekennzeichnet durch** eine Kardioversionseinheit (48), die zur Erzeugung und Abgabe von zur Kardioversion oder zur Defibrillation geeigneten Stimulationsimpulsen an definierten Zeitpunkten ausgebildet ist.

**41.** Vorrichtung nach Anspruch 40, **dadurch gekennzeichnet, daß** die Kardioversionseinheit (48) eingangsseitig mit dem Herzphasenmessfühler (24, 26, 28) verbunden ist.

**42.** Vorrichtung nach Anspruch 40 oder 41, **dadurch gekennzeichnet, daß** die Schrittmachersteuermittel (22.7) ausgangsseitig mit der Kardioversionseinheit (48) verbunden und zur Erzeugung und Abgabe eines Kardioversionsaktivierungs- oder -deaktivierungssignals ausgebildet sind.

**43.** Vorrichtung nach Anspruch 42, **dadurch gekennzeichnet, daß** die Schrittmachersteuermittel (22.7) zur Abgabe eines Triggerdeaktivierungssignals und eines Deaktivierungssignals bei Abgabe eines Kardioversionsaktivierungssignals ausgebildet sind.

**Claims**

**1.** Method for signalling an internal status of an implantable cardiac stimulation device, which status is defined by a value of a status variable, comprising the following steps:

- recording a measurement value (S12) representing the status variable;

- ascertaining the existence of the status by comparing the measurement value with a comparative value (S14);

- delivering a signal pulse with a predetermined, measurable pulse parameter, wherein the pulse parameter is uniquely associated with the status if the measurement value corresponds to the comparative value within predetermined limits (S26);

**characterised by** the following steps:

measuring the time difference of equal, predetermined phases of two successive, periodically recurring sequences of events prior to delivery of the signal pulse (S20); and then

- delivering the signal pulse prior to or after the next expiry of the measured time difference (S22, S24, S26) by a predeterminable period of time uniquely associated with the status.

**2.** Method according to claim 1, **characterised in that** the method is started as soon as a triggering signal is present in the device.

**3.** Method according to claim 2, **characterised in that** the method is carried out again if the triggering signal is still present after it has been carried out.

**4.** Method according to either claim 2 or claim 3, **characterised in that** the triggering signal is produced as long as a magnetic field with a predetermined magnetic field parameter is detected in the device.

**5.** Method according to any one of the preceding claims, **characterised in that** no time-displaced signal pulse is produced subsequently to the delivery of the time-displaced signal pulse for a predeterminable number of subsequent sequences of events (S28).

**6.** Method according to any one of the preceding claims, **characterised by** the measurement of a physical variable that is dependent on the phase of the cardiac cycle.

**7.** Method according to claim 6, **characterised by** the recording of an electrocardiogram.

**8.** Method according to any one of the preceding claims, **characterised in that** the recorded measurement value is an electrical voltage at an output of an energy storage means arranged in the device (S12).

**9.** Method according to any one of the preceding claims, **characterised in that** the delivered signal pulse is an electrical current pulse.

**10.** Heart stimulation device comprising:

- a pulse generator (12) for producing cardiac stimulation pulses;

- a stimulation electrode (16) for delivering the pulses, the stimulation electrode being connected to the pulse generator (12);

- a signal generator (22.2) for producing and delivering pulse triggering signals at defined moments in time ($t_A$), the signal generator being connected to the pulse generator (12);

- a cardiac phase measurement sensor (24, 26, 28) for recording a first measurement value which is dependent on the current phase of the cardiac cycle; and comprising

- means (22.9) for measuring time to determine a cycle duration between the occurrence of a predetermined phase (Q) of a defined first cardiac cycle (P1, P4) and the occurrence of the same phase (Q') of the directly subsequent second cardiac cycle (P2, P5), the means being connected to the cardiac phase measurement sensor (24, 26, 28);

time matching means (22.5) being provided to predetermine the period of time between the occurrence of the predetermined phase (Q') of the second cardiac cycle and the subsequent production of a pulse triggering signal, the time matching means being connected at the input side to the time measurement means (22.9) and at the output side to the signal generator (22.2), and a status measurement sensor (42) for recording a status measurement value that depends on the current value of a status variable of the device, the status measurement sensor being connected to the time matching means (22.5)

**11.** Heart stimulation device comprising:

- a pulse generator (12) for producing cardiac stimulation pulses;

- a stimulation electrode (16) for delivering the pulses, the stimulation electrode being connected to the signal generator (12);

- a signal generator (22.2) for producing and delivering pulse triggering signals at defined moments in time ($t_A$), the pulse generator being connected to the pulse generator (12);

- a cardiac phase measurement sensor (24, 26, 28) for recording a first measurement value which is dependent on the current phase of the cardiac cycle; and comprising

- means (22.9) for measuring time to determine a cycle duration between the occurrence of a predetermined phase (Q) of a defined first cardiac cycle (P1, P4) and the occurrence of the same phase (Q') of the directly subsequent second cardiac cycle (P2, P5), the means being connected to the cardiac phase measurement sensor (24, 26, 28);

time matching means (22.5) being provided to predetermine the period of time between the occurrence of the predetermined phase (Q') of the second cardiac cycle and the subsequent production of a pulse triggering signal, the time matching means being connected at the input side to the time measurement means (22.9) and at the output side to the signal generator (22.2), and a status measurement sensor (42) for recording a status measurement value that depends on the current value of a status variable of the device, the status measurement sensor being connected to the time matching means (22.5)

**12.** Device according to claim 11, **characterised in that** the status measurement sensor (42) is configured such that the status measurement value depends on the energy content of an energy storage device (40) of the device (10).

**13.** Device according to either claim 11 or claim 12, **characterised by** comparative value storage means (36.3) for receiving a comparative value for the status measurement value, the comparative value storage means being connected to the time matching means (22.5).

**14.** Device according to claim 13, **characterised by** first comparison means (44) for comparing the status measurement value to the comparative value and producing and delivering a status signal that is dependent on the comparison result, the first comparison means being connected at the input side to the status measurement sensor (42) and the comparative value storage means (36.3) and at the output side to the time matching means (22.5).

**15.** Device according to claim 14, **characterised by** a monitoring unit (22.10) for comparing the status signal to a pre-stored reference status value range and producing and delivering a waiting signal for the case that the status signal is not contained in the reference status value range, the monitoring unit being connected upstream of the time matching means (22.5) and being connected at the input side to the first comparison means (44).

**16.** Device according to any one of claims 10 to 15, **characterised in that** an activation measurement sensor (32) records an activation measurement value of an activation measurement variable, the activation measurement sensor being connected to the time matching means (22.5).

**17.** Device according to claim 16, **characterised in that** the activation measurement sensor (32) is a magnetic field sensor.

**18.** Device according to either claim 16 or claim 17, **characterised by** pattern value storage means (36.2) for recording a pattern value for the activation measurement value.

**19.** Device according to claim 18, **characterised by** second comparison means (34) for comparing the activation measurement value to the pattern value and, in the case of coincidence, for producing and delivering a triggering signal, the second comparison means being connected at the input side to the activation measurement sensor (32) and the pattern value storage means (36.2) and at the output side to the time matching means (22.5).

**20.** Device according to claims 14, 15 and 19, **characterised in that** the monitoring unit (22.10) is additionally connected at the input side to the second comparison means (34) and is also configured such that the waiting signal can be produced only when a triggering signal is present.

**21.** Device according to any one of claims 10 to 20, **characterised in that** the cardiac phase measurement sensor (24, 26, 28) records a measurement signal which is dependent on the electrical polarisation of the myocardium.

**22.** Device according to any one of claims 10 to 21, **characterised in that** the time measurement means (22.9) determines the QQ-time interval of successive cardiac cycles.

**23.** Device according to any one of claims 10 to 22, **characterised in that** the time matching means (22.5) produces the subsequent pulse triggering signal no earlier than 300 milliseconds prior to the expiry of the given cycle duration after the occurrence of the predetermined phase (Q') of the second cardiac cycle (P2, P5).

**24.** Device according to claim 23, **characterised in that** the time matching means (22.5) produces the subsequent pulse triggering signal no earlier than 200 milliseconds prior to the expiry of the given cycle duration after the occurrence of the predetermined phase (Q') of the second cardiac cycle.

**25.** Device according to any one of claims 10 to 24, **characterised in that** the time matching means (22.5) produces the subsequent pulse triggering signal no later than 50 milliseconds prior to the expiry of the given cycle duration after the occurrence of the predetermined phase of the second cardiac cycle (Q').

**26.** Device according to claim 25, **characterised in that** the time matching means (22.5) produces the subsequent pulse triggering signal no later than 100 milliseconds prior to the expiry of the given cycle duration after the occurrence of the predetermined phase (Q') of the second cardiac cycle.

**27.** Device according to any one of claims 10 to 26, **characterised by** pacemaker control means (22.7) for selectively producing an activation or deactivation signal for respectively activating or deactivating operation of the time matching means (22.5), the pacemaker control means being connected to the time matching means (22.5).

**28.** Device according to claim 27, **characterised in that** the pacemaker control means (22.7) activates or deactivates operation of the time matching means (22.5) for a predeterminable number of cardiac cycles, the pacemaker control means being connected at the input side to the cardiac phase measurement sensor (24, 26, 28).

**29.** Device according to claim 28, **characterised in that** the pacemaker control means (22.7) activates operation of the time matching means (22.5) for the duration of a cardiac cycle (P2, P5) and immediately subsequently deactivates operation of the time matching means (22.5).

**30.** Device according to claim 29, **characterised in that** the pacemaker control means (22.7) directly subsequently deactivates operation of the time matching means (22.5) for the duration of at least two cardiac cycles (P3, P4).

**31.** Device according to either claim 29 or claim 30, **characterised in that** the pacemaker control means (22.7) directly subsequently deactivates operation of the time matching means (22.5) for the duration of at most 15 cardiac cycles.

**32.** Device according to any one of claims 29 to 31, **characterised in that** the pacemaker control means (22.7) directly subsequently deactivates operation of the time matching means (22.5) for the duration of 5 cardiac cycles.

**33.** Device according to any one of claims 10 to 32, **characterised in that** a cardiac monitoring unit (22.8) compares the respectively measured cycle duration (T1, T3, T4, T6) to a reference cycle duration value range contained in a reference cycle value storage means, the cardiac monitoring unit being connected to the time measurement means (22.9) upstream of the pacemaker control means (22.7).

**34.** Device according to claim 33, **characterised in that** the cardiac monitoring unit (22.8), in the event of a deviation of the measured cycle duration (T1, T3, T4, T6) from the reference cycle duration value range, produces a therapy warning signal which is dependent on the value of the deviation.

**35.** Device according to either claim 33 or claim 34, **characterised in that** the pacemaker control means (22.7) produces and communicates a deactivation signal to the time matching means (22.5) when a therapy warning signal is received.

**36.** Device according to any one of claims 33 to 35, **characterised in that** the pacemaker control means (22.7) blocks the production of an activation signal for a defined period of time when a therapy warning signal is received.

**37.** Device according to any one of claims 10 to 36, **characterised by** a trigger unit (22.4) for producing and delivering triggering signals to the signal generator (22.2) at defined moments in time in a pacemaker therapy, the triggering signals causing the signal generator (22.2) to produce and deliver pulse triggering signals, the trigger unit being connected at the input side to the cardiac phase measurement sensor (24, 26, 28) and at the output side to the signal generator (22.2).

**38.** Device according to claim 37, **characterised in that** the pacemaker control means (22.7) is additionally connected to the trigger unit (22.4) and produces and delivers a trigger activation or deactivation signal to the trigger unit (22.4), wherein when a trigger activation signal is delivered to the trigger unit (22.4), a deactivation signal is also delivered to the time matching means (22.5) and, *vice versa*, when an activation signal is delivered to the time matching means (22.5), a trigger deactivation signal is also delivered to the trigger unit (22.4).

**39.** Device according to claim 38, **characterised in that** the pacemaker control means (22.7) produces a trigger activation signal when a therapy warning signal is present.

**40.** Device according to any one of claims 10 to 39, **characterised by** a cardioversion unit (48) for producing and delivering stimulation pulses suitable for cardioversion or defibrillation at defined moments in time.

**41.** Device according to claim 40, **characterised in that** the cardioversion unit (48) is connected at the input side to the cardiac phase measurement sensor (24, 26, 28).

**42.** Device according to either claim 40 or claim 41, **characterised in that** the pacemaker control means (22.7) is connected at the output side to the cardioversion unit (48) and produces and delivers a cardioversion activation or deactivation signal.

**43.** Device according to claim 42, **characterised in that** the pacemaker control means (22.7) delivers a trigger deactivation signal and a deactivation signal on delivery of a cardioversion activation signal.

**Revendications**

**1.** Procédé de signalisation d'un état interne d'un dispositif de stimulation cardiaque implantable défini par une valeur d'une grandeur d'état comprenant les étapes suivantes :

- réception d'une valeur de mesure représentant la grandeur d'état (S12),
- détermination de la présence de l'état par comparaison de la valeur de mesure avec une valeur de comparaison (S14),
- émission d'une impulsion de signal avec un paramètre d'impulsion mesurable prédéterminé, le paramètre d'impulsion étant clairement associé à l'état, dans le cas où la valeur de mesure correspond à la valeur de comparaison à l'intérieur de limites prédéterminées (S26),

    **caractérisé par** les étapes de :

- mesure de la différence de temps de phases identiques prédéterminées de deux séries d'événements successives, se répétant périodiquement, avant l'émission de l'impulsion de signal (S20), puis

- émission de l'impulsion de signal à un laps de temps prédéterminable et associé clairement à l'état, avant ou après la prochaine expiration de la différence de temps mesurée (S22, S24, S26).

2. Procédé selon la revendication 1, **caractérisé en ce qu'**il est mis en marche dès qu'un signal de déclenchement est présent dans le dispositif.

3. Procédé selon la revendication 2, **caractérisé en ce qu'**il est de nouveau exécuté si le signal de déclenchement est encore présent après l'exécution.

4. Procédé selon la revendication 2 ou 3, **caractérisé en ce que** le signal de déclenchement est émis aussi longtemps qu'un champ magnétique avec un paramètre de champ magnétique prédéterminé est détecté dans le dispositif.

5. Procédé selon l'une quelconque des revendications précédentes, **caractérisé en ce qu'**aucune impulsion de signal à décalage de temps n'est émise à la suite de l'émission de l'impulsion de signal à décalage de temps pour un nombre prédéterminé de séries d'événements successives (S28).

6. Procédé selon l'une quelconque des revendications précédentes, **caractérisé par** la mesure d'une grandeur physique dépendant de la phase du cycle cardiaque.

7. Procédé selon la revendication 6, **caractérisé par** l'enregistrement d'un électrocardiogramme.

8. Procédé selon l'une quelconque des revendications précédentes, **caractérisé en ce que** l'on enregistre comme valeur de mesure une tension électrique appliquée à la sortie d'un accumulateur d'énergie disposé dans le dispositif (S12).

9. Procédé selon l'une quelconque des revendications précédentes, **caractérisé en ce que** l'on émet comme impulsion de signal une impulsion de courant électrique.

10. Dispositif de stimulation cardiaque comportant

- un générateur d'impulsions (12) configuré pour la production d'impulsions adéquates pour la stimulation cardiaque,
- une électrode de stimulation (16) reliée au générateur d'impulsions (12) et configurée pour l'émission des impulsions,
- un émetteur de signaux (22.2) relié au générateur d'impulsions (12) et configuré pour la production et l'émission de signaux de déclenchement d'impulsions à des instants définis ($t_A$),

- un capteur de mesure des phases cardiaques (24, 26, 28) configuré pour l'enregistrement d'une première valeur de mesure dépendant de la phase actuelle du cycle cardiaque, et avec
- des moyens de mesure du temps (22.9) reliés au capteur de mesure des phases cardiaques (24, 26, 28) et configurés pour la détermination d'une durée de cycle entre l'entrée d'une phase prédéterminée (Q) d'un premier cycle cardiaque défini (P1, P4) et l'entrée de la même phase (Q') du deuxième cycle cardiaque (P2, P5) qui suit immédiatement,

   dans lequel

- on prévoit des moyens de synchronisation (22.5) reliés du côté de l'entrée aux moyens de mesure du temps (22.9) et du côté de la sortie à l'émetteur de signaux (22.2), qui sont configurés pour prédéterminer le laps de temps entre l'entrée de la phase prédéterminée (Q') du deuxième cycle cardiaque et la production suivante d'un signal de déclenchement d'impulsion, et
- on prévoit un capteur de mesure d'état (42) relié aux moyens de synchronisation (22.5) configuré pour l'enregistrement d'une valeur de mesure d'état qui dépend de la valeur actuelle d'une grandeur d'état du dispositif.

11. Dispositif de stimulation cardiaque comprenant

- un générateur d'impulsions (12) configuré pour la production d'impulsions adéquates pour la stimulation cardiaque,
- une électrode de stimulation (16) reliée au générateur d'impulsions (12) et configurée pour l'émission des impulsions,
- un émetteur de signaux (22.2) relié au générateur d'impulsions (12) et qui est configuré pour la production et l'émission de signaux de déclenchement d'impulsions à des instants définis ($t_A$),
- un capteur de mesure des phases cardiaques (24, 26, 28) configuré pour l'enregistrement d'une première valeur de mesure dépendant de la phase actuelle du cycle cardiaque, et avec
- des moyens de mesure du temps (22.9) reliés au capteur de mesure des phases cardiaques (24, 26, 28) et configurés pour la détermination d'une durée de cycle entre l'entrée d'une phase prédéterminée (Q) d'un premier cycle cardiaque défini (P1, P4) et l'entrée de la même phase (Q') du deuxième cycle cardiaque (P2, P5) qui suit immédiatement,

   dans lequel

- on prévoit des moyens de synchronisation (22.5) reliés du côté de l'entrée aux moyens de mesure du temps (22.9) et du côté de la sortie à l'émetteur de signaux (22.2), qui sont configurés pour prédéterminer le laps de temps entre l'entrée de la phase prédéterminée (Q') du deuxième cycle cardiaque et la production suivante d'un signal de déclenchement d'impulsion, et
- on prévoit un capteur de mesure d'état (42) relié aux moyens de synchronisation (22.5), qui est configuré pour l'enregistrement d'une valeur de mesure d'état, qui dépend de la valeur actuelle d'une grandeur d'état du dispositif.

12. Dispositif selon la revendication 11, **caractérisé en ce que** le capteur de mesure d'état (42) est configuré de sorte que la valeur de mesure d'état dépende du contenu en énergie d'un accumulateur d'énergie (40) du dispositif (10).

13. Dispositif selon la revendication 11 ou 12, **caractérisé par** une mémoire de valeurs de comparaison (36.3) reliée aux moyens de synchronisation (22.5) pour l'enregistrement d'une valeur de comparaison pour la valeur de mesure d'état.

14. Dispositif selon la revendication 13, **caractérisé par** des premiers moyens de comparaison (44) reliés du côté de l'entrée au capteur de mesure d'état (42) et à la mémoire de valeurs de comparaison (36.3) et du côté de la sortie aux moyens de synchronisation (22.5) et qui sont configurés pour la comparaison de la valeur de mesure d'état avec la valeur de comparaison ainsi que pour la production et l'émission d'un signal d'état dépendant du résultat de la comparaison.

15. Dispositif selon la revendication 14, **caractérisé par** une unité de surveillance (22.10) installée avant les moyens de synchronisation (22.5) et reliée du côté de l'entrée aux premiers moyens de comparaison (44), qui est configurée pour la comparaison du signal d'état avec un intervalle de valeur de consigne d'état mémorisé au préalable et pour la production et l'émission d'un signal de maintenance, pour le cas où le signal d'état ne serait pas contenu dans l'intervalle de valeur de consigne d'état.

16. Dispositif selon l'une quelconque des revendications 10 à 15, **caractérisé en ce qu'**on prévoit un capteur de mesure d'activation (32) relié aux moyens de synchronisation (22.5) configuré pour l'enregistrement d'une valeur de mesure d'activation d'une grandeur de mesure d'activation.

17. Dispositif selon la revendication 16, **caractérisé en ce que** le capteur de mesure d'activation (32) est un détecteur de champ magnétique.

18. Dispositif selon la revendication 16 ou 17, **caractérisé par** une mémoire de valeurs d'échantillons (36.2) pour l'enregistrement d'une valeur d'échantillon pour le capteur de mesure d'activation.

19. Dispositif selon la revendication 18, **caractérisé par** des deuxièmes moyens de comparaison (34) reliés du côté de l'entrée au capteur de mesure d'activation (32) et à la mémoire de valeurs d'échantillons (36.2) et du côté de la sortie aux moyens de synchronisation (22.5) et qui sont configurés pour la comparaison de la valeur de mesure d'activation avec la valeur d'échantillon ainsi que, en cas d'accord, pour la production et l'émission d'un signal de déclenchement.

20. Dispositif selon la revendication 14, 15 et 19, **caractérisé en ce que** l'unité de surveillance (22.10) est, de plus, reliée du côté de l'entrée aux deuxièmes moyens de comparaison (34) et est, de plus, configurée de telle façon que le signal de maintenance ne puisse être produit que s'il existe un signal de déclenchement.

21. Dispositif selon l'une quelconque des revendications 10 à 20, **caractérisé en ce que** le capteur de mesure des phases cardiaques (24, 26, 28) est configuré pour l'enregistrement d'un signal de mesure dépendant de la polarisation électrique du myocarde.

22. Dispositif selon l'une quelconque des revendications 10 à 21, **caractérisé en ce que** les moyens de mesure du temps (22.9) sont configurés pour la détermination de l'intervalle de temps QQ de cycles cardiaques successifs.

23. Dispositif selon l'une quelconque des revendications 10 à 22, **caractérisé en ce que** les moyens de synchronisation (22.5) sont configurés pour la production du signal de déclenchement d'impulsion au moins 300 millisecondes avant l'expiration de la durée de cycle déterminée après l'entrée de la phase prédéterminée (Q') du deuxième cycle cardiaque (P2, P5).

24. Dispositif selon la revendication 23, **caractérisé en ce que** les moyens de synchronisation (22.5) sont configurés pour la production du signal de déclenchement d'impulsion au moins 200 millisecondes avant l'expiration de la durée de cycle déterminée après l'entrée de la phase prédéterminée (Q') du deuxième cycle cardiaque.

25. Dispositif selon l'une quelconque des revendications 10 à 24, **caractérisé en ce que** les moyens de synchronisation (22.5) sont configurés pour la production du signal de déclenchement d'impulsion au maximum 50 millisecondes avant l'expiration de la durée de cycle déterminée après l'entrée de la phase prédéterminée du deuxième cycle cardiaque (Q').

26. Dispositif selon la revendication 25, **caractérisé en ce que** les moyens de synchronisation (22.5) sont configurés pour la production du signal de déclenchement d'impulsion au maximum 100 millisecondes avant l'expiration de la durée de cycle déterminée après l'entrée de la phase prédéterminée (Q') du deuxième cycle cardiaque.

27. Dispositif selon l'une quelconque des revendications 10 à 26, **caractérisé par** des moyens de commande de stimulateur (22.7) reliés aux moyens de synchronisation (22.5), qui sont configurés pour la production sélective d'un signal d'activation ou d'un signal de désactivation pour l'activation ou la désactivation du fonctionnement des moyens de synchronisation (22.5).

28. Dispositif selon la revendication 27, **caractérisé en ce que** les moyens de commande de stimulateur (22.7) sont reliés du côté de l'entrée au capteur de mesure des phases cardiaques (24, 26, 28) et sont configurés pour l'activation ou la désactivation du fonctionnement des moyens de synchronisation (22.5) pour un nombre prédéterminable de cycles cardiaques.

29. Dispositif selon la revendication 28, **caractérisé en ce que** les moyens de commande de stimulateur (22.7) sont configurés pour l'activation du fonctionnement des moyens de synchronisation (22.5) pour la durée d'un cycle cardiaque (P2, P5) et pour la désactivation consécutive immédiate du fonctionnement des moyens de synchronisation (22.5).

30. Dispositif selon la revendication 29, **caractérisé en ce que** les moyens de commande de stimulateur (22.7) sont configurés pour la désactivation consécutive immédiate du fonctionnement des moyens de synchronisation (22.5) pour la durée d'au moins deux cycles cardiaques (P3, P4).

31. Dispositif selon la revendication 29 ou 30, **caractérisé en ce que** les moyens de commande de stimulateur (22.7) sont configurés pour la désactivation consécutive immédiate du fonctionnement des moyens de synchronisation (22.5) pour la durée de 15 cycles cardiaques au maximum.

32. Dispositif selon l'une quelconque des revendications 29 à 31, **caractérisé en ce que** les moyens de commande de stimulateur (22.7) sont configurés pour la désactivation consécutive immédiate du fonctionnement des moyens de synchronisation (22.5) pour la durée de 5 cycles cardiaques.

33. Dispositif selon l'une quelconque des revendications 10 à 32, **caractérisé en ce qu'**une unité de surveillance cardiaque (22.8) reliée aux moyens de mesure du temps (22.9) est installée avant les moyens de commande de stimulateur (22.7), et est configurée pour la comparaison de la durée de cycle respectivement mesurée (T1, T3, T4, T6) avec un intervalle de valeur de consigne de la durée de cycle contenu dans une mémoire de valeurs de consigne de cycle.

34. Dispositif selon la revendication 33, **caractérisé en ce que** l'unité de surveillance cardiaque (22.8) est configurée pour la production d'un signal d'alerte de thérapie dépendant de la valeur de l'écart, dans le cas où il existe un écart entre la durée de cycle mesurée (T1, T3, T4, T6) et l'intervalle de valeur de consigne de la durée de cycle.

35. Dispositif selon la revendication 33 ou 34, **caractérisé en ce que** les moyens de commande de stimulateur (22.7) sont configurés pour la production et la transmission d'un signal de désactivation aux moyens de synchronisation (22.5) lors de la réception d'un signal d'alerte de thérapie.

36. Dispositif selon l'une quelconque des revendications 33 à 35, **caractérisé en ce que** les moyens de commande de stimulateur (22.7) sont configurés pour le blocage de la production d'un signal d'activation pour une période définie lors de la réception d'un signal d'alerte de thérapie.

37. Dispositif selon l'une quelconque des revendications 10 à 36, **caractérisé par** une unité de déclenchement (22.4) reliée du côté de l'entrée au capteur de mesure des phases cardiaques (24, 26, 28) et du côté de la sortie à l'émetteur de signaux (22.2), qui est configurée pour la production et l'émission de signaux de déclenchement vers l'émetteur de signaux (22.2) à des instants définis dans le cadre d'une thérapie par stimulateur, les signaux de déclenchement conduisant l'émetteur de signaux (22.2) à produire et à émettre des signaux de déclenchement d'impulsions.

38. Dispositif selon la revendication 37, **caractérisé en ce que** les moyens de commande de stimulateur (22.7) sont en plus reliés à l'unité de déclenchement (22.4) et sont configurés pour la production et l'émission d'un signal d'activation de déclenchement ou d'un signal de désactivation de déclenchement vers l'unité de déclenchement (22.4), dans lequel se produit également l'émission d'un signal de désactivation vers les moyens de synchronisation (22.5) lors de l'émission d'un signal d'activation de déclenchement vers l'unité de déclenchement (22.4), et vice-versa, dans lequel se produit également l'émission d'un signal de désactivation de déclenchement vers l'unité de déclenchement (22.4) lors de l'émission d'un signal d'activation vers les moyens de synchronisation (22.5).

**39.** Dispositif selon la revendication 38, **caractérisé en ce que** les moyens de commande de stimulateur (22.7) sont configurés pour la production d'un signal d'activation de déclenchement lors de la présence d'un signal d'alerte de thérapie.

**40.** Dispositif selon l'une quelconque des revendications 10 à 39, **caractérisé par** une unité de cardioversion (48) configurée pour la production et l'émission d'impulsions de stimulation appropriées pour la cardioversion ou la défibrillation à des instants définis.

**41.** Dispositif selon la revendication 40, **caractérisé en ce que** l'unité de cardioversion (48) est reliée du côté de l'entrée au capteur de mesure des phases cardiaques (24, 26, 28).

**42.** Dispositif selon la revendication 40 ou 41, **caractérisé en ce que** les moyens de commande de stimulateur (22.7) sont reliés du côté de la sortie à l'unité de cardioversion (48) et sont configurés pour la production et l'émission d'un signal d'activation ou de désactivation de cardioversion.

**43.** Dispositif selon la revendication 42, **caractérisé en ce que** les moyens de commande de stimulateur (22.7) sont configurés pour l'émission d'un signal de désactivation de déclenchement et d'un signal de désactivation lors de l'émission d'un signal d'activation de cardioversion.

START — S10

Ausgangsspannung
der Batterie messen — S12

Ja

Meßwert
=
Sollwert? — S14

Nein

S18

Ende ← Nein — externe Abfrage? — S16

Ja

Herz-Periodendauer T
messen
$T = t(Q') - t(Q)$ — S20

Auslösezeitpunkt $t_A$
bestimmen
$t_A = t(Q') + T - 100ms$ — S22

Nein

$t = t_A$? — S24

Ja

Signalimpuls auslösen — S26

n Q - Phasen abwarten — S28

Fig. 1

Fig. 2

EP 1 161 966 B1

Fig. 3

Fig. 4